# EUROPEAN PATENT SPECIFICATION

(11) **EP 4 104 176 B1**
(45) Date of publication and mention of the grant of the patent: **26.08.2026**
(21) Application number: 21711091.5
(22) Date of filing: 12.02.2021
(51) Int. Cl.: G16B 20/20

(54) **METHODS AND SYSTEMS FOR DETERMINING FUSION EVENTS**
VERFAHREN UND SYSTEME ZUM BESTIMMEN VON FUSIONSEREIGNISSEN
PROCÉDÉS ET SYSTÈMES POUR DÉTERMINER DES ÉVÉNEMENTS DE FUSION

(30) Priority: 14.02.2020 US 202062976884 P
(43) Date of publication of application: 21.12.2022
(73) Proprietor: Guardant Health, Inc., Palo Alto, CA 94304 (US)
(72) Inventor: GNERRE, Sante, Redwood City, CA 94063 (US)
(74) Representative: Carpmaels & Ransford LLP
(86) International application number: PCT/US2021/017995
(87) International publication number: WO 2021/163592

(56) References cited:
- WO-A1-2018/152542
- ANONYMOUS: "Archer Analysis 4.1 User Manual", 26 August 2016 (2016-08-26), XP093286596, Retrieved from the Internet <URL:https://www.manuallib.com/download/2023-10-18/Archer %20Analysis%204.1%20User%20Manual.pdf>
- DANIEL L. CAMERON ET AL: "GRIDSS: sensitive and specific genomic rearrangement detection using positional de Bruijn graph assembly", GENOME RESEARCH, vol. 27, no. 12, 2 November 2017 (2017-11-02), US, pages 2050 - 2060, XP055522516, ISSN: 1088-9051, DOI: 10.1101/gr.222109.117
- GAWRONSKI ALEXANDER R ET AL: "Structural variation and fusion detection using targeted sequencing data from circulating cell free DNA", NUCLEIC ACIDS RESEARCH, vol. 47, no. 7, 23 April 2019 (2019-04-23), pages e38 - e38, XP055802947, ISSN: 0305-1048, Retrieved from the Internet <URL:https://academic.oup.com/nar/article-pdf/47/7/e38/28467885/gkz067.pdf> DOI: 10.1093/nar/gkz067

## Description

### BACKGROUND

Cancer is one of the leading causes of deaths in the world and a class of heterogeneous complex diseases with multiple genes in diverse pathways involved in its initiation, uncontrolled growth, invasion, and metastasis. One hallmark of cancer is genetic instability that can result in chromosomal translocation, insertion, duplication, deletion, and inversion. These genetic alterations often cause genes fusions, which in turn are transcribed into fusion mRNAs or fusion transcripts. However, *de novo* detection of such fusion events can be challenging, especially if high specificity is required, as technical artifacts introduced both at the assay level, and at the analytical level, can result in false positives. This is exacerbated if the input data contains sequences generated by assays with ultra-deep coverage.

Thus, there is a need for improved systems and methods for detecting fusion events that significantly increases the specificity without negatively impacting the overall sensitivity. Therefore, it is an object of the invention to provide computer-implemented systems and methods that have improved capability to detect fusion events through *de novo* assembly of input sequence reads before calling fusion events.

Cameron et al. (Genome Res. 2017; 27(12):2050-2060) discuss the structural variant caller 'GRIDSS'; and Gawroński et al. (Nucleic Acids Res. 2019; 47(7):e38) discuss the structural variant caller 'SViCT'. WO 2018/152542 (Univ Leland Stanford Junior [US]) discusses the *"accurate and sensitive unveiling of chimeric biomolecule sequences and applications thereof*" (title).

### SUMMARY

It is to be understood that both the following general description and the following detailed description are exemplary and explanatory only and are not restrictive. Methods, systems, and apparatuses for determining fusion events are described herein and set out in the claims.

Methods according to the claimed invention are computer-implemented and comprise aligning a plurality of sequence reads to a reference sequence, determining one or more breakpoints in an alignment of a plurality of sequence reads of the plurality of sequence reads to the reference sequence, identifying any sequence reads associated with the one or more breakpoints in the alignment as candidate fusion sequence reads, determining candidate fusion sequence reads associated with common breakpoints of one or more breakpoints, grouping the candidate fusion sequence reads based on one or more common breakpoints, assembling the candidate fusion sequence reads in the groups into one or more contigs, aligning the contigs from the groups to the reference sequence, determining, based on the alignments of the contigs from the groups, one or more candidate fusion events as set out in the claims, applying one or more criteria to the one or more candidate fusion events, and determining, based on applying the one or more criteria to the one or more candidate fusion events, one or more fusion events.

In certain embodiments, identifying any sequence reads associated with the one or more breakpoints in the alignment as candidate fusion sequence reads comprises discarding alignments that are logical. In certain embodiments, determining candidate fusion sequence reads associated with common breakpoints of one or more breakpoints comprises determining that at least two candidate fusion sequence reads comprise a breakpoint in a same chromosome and at a same orientation. In certain embodiments, determining candidate fusion sequence reads associated with common breakpoints of one or more breakpoints comprises determining that at least two candidate fusion sequence reads comprise a breakpoint at a same position. In certain embodiments, determining candidate fusion sequence reads associated with common breakpoints of one or more breakpoints comprises determining that at least two candidate fusion sequence reads comprise a breakpoint within a threshold number of bases from a position. In certain embodiments, determining candidate fusion sequence reads associated with common breakpoints of one or more breakpoints comprises determining that at least two candidate fusion sequence reads comprise a plurality of breakpoints in a same chromosome and at a same orientation. In certain embodiments, determining candidate fusion sequence reads associated with common breakpoints of one or more breakpoints comprises determining that at least two candidate fusion sequence reads comprise a plurality of breakpoints at same positions. In certain embodiments, determining candidate fusion sequence reads associated with common breakpoints of one or more breakpoints comprises determining that at least two candidate fusion sequence reads each comprise a plurality of breakpoints within a threshold number of bases from a plurality of positions.

In certain embodiments, grouping the candidate fusion sequence reads based on one or more common breakpoints comprises generating a de Bruijn graph for the groups. In certain embodiments, assembling the candidate fusion sequence reads in the groups into one or more contigs comprises linearizing the de Bruijn graphs to generate a contig for the groups. In certain embodiments, assembling the candidate fusion sequence reads in the groups into one or more contigs comprises performing one or more error correction procedures. In certain embodiments, the one or more error correction procedures comprises resolving mismatches between candidate fusion sequence reads and the reference sequence. In certain embodiments, the one or more error correction procedures comprises inserting padding between at least two candidate fusion sequence reads. In certain embodiments, the one or more error correction procedures comprises discarding one or more candidate fusion sequence reads having an unaligned portion that exceeds a threshold.

According to the claimed invention, determining, based on the alignments of the contigs from the groups, one or more candidate fusion events comprises applying a spread test and may further comprise applying a footprint test. In certain embodiments, applying the footprint test comprises determining that a threshold number of families of candidate fusion sequence reads that support the contig span the breakpoint(s).

In certain embodiments, applying one or more criteria to the one or more candidate fusion events comprises: determining, for the candidate fusion events, a distance between a breakpoint of the one or more aligned contigs and a location of at least one probe of a panel; and discarding any candidate fusion event associated with an aligned contig of the one or more contigs containing no breakpoint with a distance from the location of at least one probe of a panel less than a threshold. In certain embodiments, applying one or more criteria to the one or more candidate fusion events comprises: determining one or more genes of interest; and discarding any candidate fusion event associated with an aligned contig of the one or more contigs containing no breakpoint that is associated with the one or more genes of interest. In certain embodiments, applying one or more criteria to the one or more candidate fusion events comprises: determining, for the candidate fusion events, that a breakpoint of the one or more aligned contigs is a deletion; and discarding any candidate fusion event associated with an aligned contig of the one or more contigs comprising a deletion located within a number of bases away from another deletion. In certain embodiments, applying one or more criteria to the one or more candidate fusion events comprises: determining, for the candidate fusion events, that a breakpoint of the one or more aligned contigs is a deletion; and discarding any candidate fusion event associated with an aligned contig of the one or more contigs comprising a deletion comprising a number of bases less than a threshold. In certain embodiments, applying one or more criteria to the one or more candidate fusion events comprises: discarding any candidate fusion event associated with an aligned contig of the one or more contigs comprising an insertion or a deletion that is completely embedded in an intronic region. In certain embodiments, applying one or more criteria to the one or more candidate fusion events comprises: determining, for the candidate fusion event, for the one or more aligned contigs, a ratio of molecules to reads; and discarding any candidate fusion event associated with an aligned contig of the one or more contig that is associated with a ratio of molecules to reads greater than a threshold and that is not associated with a double stranded supporting molecule. In certain embodiments, applying one or more criteria to the one or more candidate fusion events comprises: determining, for the candidate fusion event, for the pairs of breakpoints of the one or more aligned contigs, a sequence abutting the breakpoints of the pair of breakpoints; aligning the sequences abutting the breakpoints of the pair of breakpoints; determining an alignment score for the alignment of the sequences abutting the breakpoints of the pair of breakpoints; and discarding any candidate fusion event associated with an aligned contig of the one or more contigs based on the alignment score exceeding a threshold. In certain embodiments, applying one or more criteria to the one or more candidate fusion events comprises: determining, for the candidate fusion events, for the pairs of breakpoints of the one or more aligned contigs, a sequence centered on the breakpoints of the pair of breakpoints; aligning the sequences centered around the breakpoints against each other; determining an alignment score for the alignment of the sequences centered around the breakpoints; and discarding any candidate fusion event associated with an aligned contig of the one or more contigs based on the alignment score exceeding a threshold.

In some embodiments, the results of the systems and methods disclosed herein are used as an input to generate a report. The report may be in a paper or electronic format. For example the fusion events as determined by the methods and systems disclosed herein can be displayed directly in such a report. Alternatively or additionally, diagnostic information or therapeutic recommendations based on the determination of the fusion events can be included in the report.

The various steps of the methods disclosed herein, or steps carried out by the systems disclosed herein, may be carried out at the same or different times, in the same or different geographical locations, e.g. countries, and/or by the same or different people.

The disclosed methods can be useful for the treatment of a subject comprising administering one or more therapeutics to a subject, wherein the subject has been determined, using the disclosed methods of determining a fusion event, to have a fusion event. Treating a subject may comprise administering a different therapeutic to a subject than one previously administered, wherein the subject has been determined, using the disclosed methods of determining a fusion event, to have a fusion event. Treating a subject may comprise discontinuing the administration of a therapeutic to a subject, wherein the subject has been determined, using the disclosed methods of determining a fusion event, to have a fusion event.

Additional advantages will be set forth in part in the description which follows or may be learned by practice. The advantages will be realized and attained by means of the elements and combinations particularly pointed out in the appended claims.

### BRIEF DESCRIPTION OF THE DRAWINGS

The accompanying drawings, which are incorporated in and constitute a part of the present description serve to explain the principles of the methods and systems described herein:
Figure 1 shows an example method.
Figures 2A-2C show example stitching and trimming processes for generating a fragment.
Figure 3 shows an example artifact from a stitching process.
Figure 4 shows an example method.
Figure 5 shows an example breakpoint.
Figure 6 shows selection of candidate fusion sequence reads.
Figure 7 shows identification of common breakpoints between two candidate fusion sequence reads.
Figure 8 shows identification of common breakpoints between two candidate fusion sequence reads.
Figure 9A-B shows minimal examples of a de Bruijn graph and a compact de Bruijn graph.
Figure 10 shows an example use of an adjacency list for each vertex of a graph data structure.
Figure 11 shows an example use of an adjacency list for each vertex and edge of a graph data structure.
Figure 12 shows an error correction procedure.
Figure 13 shows an error correction procedure.
Figure 14 shows an error correction procedure.
Figure 15 shows an error correction procedure.
Figure 16 shows a determination of a candidate fusion event.
Figure 17 shows a determination of a candidate fusion event.
Figure 18 shows FGFR2/3 fusion partner prevalence in broad cancer cohort. Frequency of FGFR2 and FGFR3 fusion partners detected in broad cancer cohort. IGR: intergenic region.
FGFR2 as a partner gene to itself represents long deletions or insertions.
Figure 19 shows FGFR3 fusion partner prevalence in advanced urothelial cancer (aUC). A number of aUC patients with FGFR3 fusions were detected by partner gene. IGR: intergenic region. FGFR3 as a partner gene to itself represents long deletions or insertions.
Figure 20 shows mutations co-occurring with FGFR2/3 fusions in broad cancer cohort Mutations occurring in at least 3 FGFR2 or FGFR3-fusion positive patients in broad cancer cohort shown. Variants with triangles show significant enrichment in the fusion-positive population (▼ p < 1e-4, ▼ ▼ p < 1e-10, chi2 test, Bonferroni correction).
Figure 21 shows an example computing device.
Figure 22 shows an example method.
Figure 23 shows an example method.

### DETAILED DESCRIPTION

As used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another configuration includes from the one particular value and/or to the other particular value. Similarly, when values are expressed as approximations, by use of the antecedent "about," it will be understood that the particular value forms another configuration. It will be further understood that the endpoints of each of the ranges are significant both in relation to the other endpoint, and independently of the other endpoint.

"Optional" or "optionally" means that the subsequently described event or circumstance may or may not occur, and that the description includes cases where said event or circumstance occurs and cases where it does not.

Throughout the description and claims of this specification, the word "comprise" and variations of the word, such as "comprising" and "comprises," means "including but not limited to," and is not intended to exclude, for example, other components, integers or steps. "Exemplary" means "an example of" and is not intended to convey an indication of a preferred or ideal configuration. "Such as" is not used in a restrictive sense, but for explanatory purposes.

The term "subject" may refer to an animal, such as a mammalian species (preferably human) or avian (e.g., bird) species. More specifically, a subject can be a vertebrate, e.g., a mammal such as a mouse, a primate, a simian or a human. Animals include farm animals, sport animals, and pets. A subject can be a healthy individual, an individual that has symptoms or signs or is suspected of having a disease or a predisposition to the disease, or an individual that is in need of therapy or suspected of needing therapy. In some embodiments, the subject is human, such as a human who has, or is suspected of having, cancer.

The phrase "cell-free nucleic acid" can be referred to as non-encapsulated nucleic acid sourced from a bodily fluid (e.g., blood, urine, CSF, etc.) from a subject. Cell-free nucleic acids include DNA (cfDNA), RNA (cfRNA), and hybrids thereof, including genomic DNA, mitochondrial DNA, circulating DNA, siRNA, miRNA, circulating RNA (cRNA), tRNA, rRNA, small nucleolar RNA (snoRNA), Piwi-interacting RNA (piRNA), long non-coding RNA (long ncRNA), or fragments of any of these. Cell-free nucleic acids can be double-stranded, single-stranded, or partially double- and single-stranded. A cell-free nucleic acid can be released into bodily fluid through secretion or cell death processes, e.g., cellular necrosis and apoptosis. Some cell-free nucleic acids are released into bodily fluid from cancer cells e.g., circulating tumor DNA (ctDNA). Others are released from healthy cells. ctDNA can be non-encapsulated tumor-derived fragmented DNA. Cell-free fetal DNA (cffDNA) is fetal DNA circulating freely in the maternal blood stream. A cell-free nucleic acid can have one or more associated epigenetic modifications, for example, can be acetylated, 5-methylated, ubiquitylated, phosphorylated, sumoylated, ribosylated, and/or citrullinated. In some embodiments, cell-free nucleic acid is cfDNA, which usually includes double-stranded cfDNA.

The term "alignment," "aligning," and the like may refer to arranging sequences of DNA or RNA to identify regions of similarity. Similarity may be related to functional, structural, and/or evolutionary relationships between the sequences. Alignment of DNA sequences involves alignment of genomic DNA of one sequence to genomic DNA of at least one other sequence. Such alignment may exclude non-genomic DNA, such as a molecular barcode, padding bases, and the like. For example, genomic DNA of a sequence read may be aligned to genomic DNA of a reference DNA sequence, excluding any molecular tag that may be attached to the sequence read.

As used herein, recitation that nucleotides "correspond to" nucleotides in a sequence refers to nucleotides identified upon alignment with the sequence to maximize identity using a standard alignment algorithm, such as the GAP algorithm.

As used herein, "sequence identity," "sequence homology," or "identity" refers to the number of identical or similar nucleotide bases in an alignment between two or more polynucleotide sequences. In one non- limiting example, "at least 90% identical to" refers to percent identities from 90 to 100% relative to the reference polynucleotide. Identity at a level of 90% or more is indicative of the fact that, assuming for exemplification purposes a test and reference polynucleotide length of 100 nucleotides are compared, no more than 10% (i.e., 10 out of 100) of nucleotides in the test polynucleotide differs from that of the reference polynucleotide. Such differences can be represented as point mutations randomly distributed over the entire length of a nucleotide sequence or they can be clustered in one or more locations of varying length up to the maximum allowable, e.g., 10/100 nucleotide difference (approximately 90% identity). Differences are defined as nucleic acid substitutions, insertions or deletions.

Sequence identity can be determined by sequence alignment of nucleic acid sequences to identify regions of similarity or identity. For purposes herein, sequence identity is generally determined by alignment to identify identical bases. The alignment can be local or global. Matches, mismatches and gaps can be identified between compared sequences. Gaps are null nucleotides inserted between the bases of aligned sequences so that identical or similar characters are aligned. Generally, there can be internal and terminal gaps. Sequence identity can be determined by taking into account gaps as the number of identical bases/length of the shortest sequence x 100. When using gap penalties, sequence identity can be determined with no penalty for end gaps (e.g., terminal gaps are not penalized). Alternatively, sequence identity can be determined without taking into account gaps as the number of identical positions/length of the total aligned sequence x 100.

As used herein, a "global alignment" is an alignment that aligns two sequences from beginning to end, aligning each base in each sequence only once. An alignment is produced regardless of whether or not there is similarity or identity between the sequences. For example, 50% sequence identity based on "global alignment" means that in an alignment of the full sequence of two compared sequences each of 100 nucleotides in length, 50% of the bases are the same. It is understood that global alignment also can be used in determining sequence identity even when the length of the aligned sequences is not the same. The differences in the terminal ends of the sequences will be taken into account in determining sequence identity, unless the "no penalty for end gaps" is selected. Generally, a global alignment is used on sequences that share significant similarity over most of their length. Exemplary algorithms for performing global alignment include the Needleman-Wunsch algorithm (Needleman et al. J. Mol. Biol. 48: 443 (1970). Exemplary programs for performing global alignment are publicly available and include the Global Sequence Alignment Tool available at the National Center for Biotechnology Information (NCBI) website (ncbi.nlm.nih.gov/), and the program available at deepc2.psi.iastate.edu/aat/align/align.html.

As used herein, a "local alignment" is an alignment that aligns two sequences, but only aligns those portions of the sequences that share similarity or identity. Hence, a local alignment determines if sub-segments of one sequence are present in another sequence. If there is no similarity, no alignment will be returned. Local alignment algorithms include BLAST or Smith- Waterman algorithm (Adv. Appl. Math. 2: 482 (1981)). For example, 50% sequence identity based on "local alignment" means that in an alignment of the full sequence of two compared sequences of any length, a region of similarity or identity of 100 nucleotides in length has 50% of the bases that are the same in the region of similarity or identity.

The phrase "nucleic acid tag" may refer to a short nucleic acid (e.g., less than 500, 100, 50, or 10 nucleotides long), used to label nucleic acid molecules to distinguish nucleic acids from different samples (e.g., representing a sample index), or different nucleic acid molecules in the same sample (e.g., representing a molecular barcode), of different types, or which have undergone different processing. Tags can be single stranded, double-stranded or at least partially double-stranded. Tags can have the same length or varied lengths. Tags can be blunt-end or have an overhang. Tags can be attached to one end or both ends of the nucleic acids. Nucleic acid tags can be decoded to reveal information such as the sample of origin, form or processing of a nucleic acid. Tags can be used to allow pooling and parallel processing of multiple samples comprising nucleic acids bearing different molecular barcodes and/or sample indexes with the nucleic acids subsequently being deconvolved by reading the molecular barcodes. Additionally or alternatively, nucleic acid tags can be used to distinguish different molecules in the same sample (i.e., molecular barcode). This includes both uniquely tagging different molecules in the sample, or non-uniquely tagging the molecules in the sample. In the case of non-unique tagging, a limited number of different tags may be used to tag molecules such that different molecules can be distinguished based on their start and/or stop position where they map on a reference genome (i.e., genomic coordinates) in combination with at least one tag. Typically then, a sufficient number of different tags are used such that there is a low probability (e.g. <10%, <5%, <1%, or <0.1%) that any two molecules having the same start/stop also have the same tag. Some tags include multiple identifiers to label samples, forms of molecule within a sample, and molecules within a form having the same start and stop points. Such tags can exist in the form A1i, wherein the letter indicates a sample type, the Arabic number indicates a form of molecule within a sample, and the Roman numeral indicates a molecule within a form.

The term "adapter" refers to a short nucleic acid (e.g., less than 500, 100, or 50 nucleotides long) usually at least partly double-stranded for linkage to either or both ends of a sample nucleic acid molecule. Adapters can include primer binding sites to permit amplification of a nucleic acid molecule flanked by adapters at both ends, and/or a sequencing primer binding site, including primer binding sites for next generation sequencing (NGS). Adapters can also include binding sites for capture probes, such as an oligonucleotide attached to a flow cell support. Adapters can also include a tag as described above. Tags are preferably positioned relative to primer and sequencing primer binding sites, such that a tag is included in amplicons and sequencing reads of a nucleic acid molecule. Adapters of the same or different sequences can be linked to the respective ends of a nucleic acid molecule. Sometimes adapters of the same sequence are linked to the respective ends except that the barcode is different. A preferred adapter is a Y-shaped adapter in which one end is blunt ended or tailed, for joining to a nucleic acid molecule, which is also blunt ended or tailed with one or more complementary nucleotides. Another preferred adapter is a bell-shaped adapter, likewise with a blunt or tailed end for joining to a nucleic acid to be analyzed.

As used herein, the terms "sequencing" or "sequencer" refer to any of a number of technologies used to determine the sequence of a biomolecule, e.g., a nucleic acid such as DNA or RNA. Exemplary sequencing methods include, but are not limited to, targeted sequencing, single molecule real-time sequencing, exon sequencing, electron microscopy-based sequencing, panel sequencing, transistor-mediated sequencing, direct sequencing, random shotgun sequencing, Sanger dideoxy termination sequencing, whole-genome sequencing, sequencing by hybridization, pyrosequencing, duplex sequencing, cycle sequencing, single-base extension sequencing, solid-phase sequencing, high-throughput sequencing, massively parallel signature sequencing, emulsion PCR, co-amplification at lower denaturation temperature-PCR (COLD-PCR), multiplex PCR, sequencing by reversible dye terminator, paired-end sequencing, near-term sequencing, exonuclease sequencing, sequencing by ligation, short-read sequencing, single-molecule sequencing, sequencing-by-synthesis, real-time sequencing, reverse-terminator sequencing, nanopore sequencing, 454 sequencing, Solexa Genome Analyzer sequencing, SOLiD^{™} sequencing, MS-PET sequencing, and a combination thereof. In some embodiments, sequencing can be performed by a gene analyzer such as, for example, gene analyzers commercially available from Illumina or Applied Biosystems.

The phrase "next generation sequencing" or NGS refers to sequencing technologies having increased throughput as compared to traditional Sanger- and capillary electrophoresis-based approaches, for example, with the ability to generate hundreds of thousands of relatively small sequence reads at a time. Some examples of next generation sequencing techniques include, but are not limited to, sequencing by synthesis, sequencing by ligation, and sequencing by hybridization.

The term "DNA (deoxyribonucleic acid)" refers to a chain of nucleotides comprising deoxyribonucleosides that each comprise one of four nucleobases, namely, adenine (A), thymine (T), cytosine (C), and guanine (G). The term "RNA (ribonucleic acid)" refers to a chain of nucleotides comprising four types of ribonucleosides that each comprise one of four nucleobases, namely; A, uracil (U), G, and C. Certain pairs of nucleotides specifically bind to one another in a complementary fashion (called complementary base pairing). In DNA, adenine (A) pairs with thymine (T) and cytosine (C) pairs with guanine (G). In RNA, adenine (A) pairs with uracil (U) and cytosine (C) pairs with guanine (G). When a first nucleic acid strand binds to a second nucleic acid strand made up of nucleotides that are complementary to those in the first strand, the two strands bind to form a double strand. As used herein, "nucleic acid sequencing data," "nucleic acid sequencing information," "nucleic acid sequence," "nucleotide sequence", "genomic sequence," "genetic sequence," or "fragment sequence," or "nucleic acid sequencing read" denotes any information or data that is indicative of the order of the nucleotide bases (e.g., adenine, guanine, cytosine, and thymine or uracil) in a molecule (e.g., a whole genome, whole transcriptome, exome, oligonucleotide, polynucleotide, or fragment) of a nucleic acid such as DNA or RNA. It should be understood that the present teachings contemplate sequence information obtained using all available varieties of techniques, platforms or technologies, including, but not limited to: capillary electrophoresis, microarrays, ligation-based systems, polymerase-based systems, hybridization-based systems, direct or indirect nucleotide identification systems, pyrosequencing, ion- or pH-based detection systems, and electronic signature-based systems.

A "polynucleotide", "nucleic acid", "nucleic acid molecule", or "oligonucleotide" refers to a linear polymer of nucleosides (including deoxyribonucleosides, ribonucleosides, or analogs thereof) joined by internucleosidic linkages. Typically, a polynucleotide comprises at least three nucleosides. Oligonucleotides often range in size from a few monomeric units, e.g. 3-4, to hundreds of monomeric units. Whenever a polynucleotide is represented by a sequence of letters, such as "ATGCCTG," it will be understood that the nucleotides are in 5'-3' order from left to right and that "A" denotes adenosine, "C" denotes cytosine, "G" denotes guanosine, and "T" denotes thymidine, unless otherwise noted. The letters A, C, G, and T may be used to refer to the bases themselves, to nucleosides, or to nucleotides comprising the bases, as is standard in the art.

The phrase "reference sequence" refers to a known sequence used for purposes of comparison with experimentally determined sequences. For example, a known sequence can be an entire genome, a chromosome, or any segment thereof. A reference typically includes at least 20, 50, 100, 200, 250, 300, 350, 400, 450, 500, 1000, or more nucleotides. A reference sequence can align with a single contiguous sequence of a genome or chromosome or can include non-contiguous segments aligning with different regions of a genome or chromosome. In some embodiments, the reference sequence is a human genome. Reference human genomes include, e.g., hG19 and hG38.

The phrase "biological sample" as used herein, generally refers to a tissue or fluid sample derived from a subject. A biological sample may be directly obtained from the subject. The biological sample may be or may include one or more nucleic acid molecules, such as deoxyribonucleic acid (DNA) or ribonucleic acid (RNA) molecules. The biological sample can be derived from any organ, tissue or biological fluid. A biological sample can comprise, for example, a bodily fluid or a solid tissue sample. An example of a solid tissue sample is a tumor sample, e.g., from a solid tumor biopsy. Bodily fluids include, for example, blood, serum, plasma, tumor cells, saliva, urine, lymphatic fluid, prostatic fluid, seminal fluid, milk, sputum, stool, tears, and derivatives of these. In some embodiments, the biological sample is, or is derived from, blood.

The phrase "fusion sequence read" in the context of nucleic acid sequence information refers to a sequencing read that includes sub-sequences that map to different non-contiguous regions or loci of a given reference sequence. A "candidate fusion sequence read" is a sequence read that may be a fusion sequence read. In certain embodiments, for example, a first sub-sequence of a given fusion sequence read maps to a first exon of a given gene of a reference sequence, while a second sub-sequence of that given fusion sequence read maps to a second exon of the same gene of the reference sequence, which first and second exons are separated by an intervening intron of the same gene of the reference sequence. In some of these embodiments, such a fusion sequence read is indicative of the presence of an intragenic fusion in the genome of a subject from whom the given fusion sequence read was obtained. In other exemplary embodiments, a first sub-sequence of a given fusion sequence read maps to an exon of a first gene of a reference sequence, while a second sub-sequence of that given fusion sequence read maps to an exon of a different second gene of the reference sequence, which exons are non-contiguous with one another in the reference sequence. In some of these embodiments, such a fusion sequence read is indicative of the presence of an intergenic fusion in the genome of a subject from whom the given fusion sequence read was obtained.

The term "sequence reads" refers to nucleotide sequences read from a sample obtained from an individual. Sequence reads can be obtained through various methods known in the art.

The term "breakpoint" in the context of a nucleic acid fusion molecule or a corresponding sequencing read refers to a terminal nucleotide position at a junction between fused sub-sequences of the nucleic acid fusion or represented in the corresponding sequencing read. For example, a given split sequence read may include a first sub-sequence that is contiguous with, and 5' to, a second sub-sequence in that split sequence read in which the first sub-sequence maps to a first locus in a reference sequence that is non-contiguous with a second locus in that reference sequence to which the second sub-sequence maps. In this example, the first sub-sequence of the split sequence read includes a breakpoint at its 3' terminal nucleotide, while the second sub-sequence of the split sequence read includes a breakpoint at its 5' terminal nucleotide. In certain applications, breakpoints such as these are referred to as a "breakpoint pair."

The term "fusion event" refers to a fusion between two separate genes at a particular location. Example causes of a fusion event include a translocation, interstitial deletion, or chromosomal inversion event.

The term "abfusion," *"de novo* fusion caller," "fusion caller," or *"de novo* method" refers to the fusion caller, either DNA or RNA fusion caller, that identifies fusion events *de novo,* that is, without prior knowledge such as can be obtained from a database of previously known gene fusion events.

The phrase "about" or "approximately" as applied to one or more values or elements of interest, refers to a value or element that is similar to a stated reference value or element. In certain embodiments, the term "about" or "approximately" refers to a range of values or elements that falls within 25%, 20%, 19%, 18%, 17%, 16%, 15%, 14%, 13%, 12%, 11%, 10%, 9%, 8%, 7%, 6%, 5%, 4%, 3%, 2%, 1%, or less in either direction (greater than or less than) of the stated reference value or element unless otherwise stated or otherwise evident from the context (except where such number would exceed 100% of a possible value or element).

It is understood that when combinations, subsets, interactions, groups, etc. of components are described that, while specific reference of each various individual and collective combinations and permutations of these may not be explicitly described, each is specifically contemplated and described herein. This applies to all parts of this application including, but not limited to, steps in described methods. Thus, if there are a variety of additional steps that may be performed it is understood that each of these additional steps may be performed with any specific configuration or combination of configurations of the described methods.

As will be appreciated by one skilled in the art, hardware, software, or a combination of software and hardware may be implemented. Furthermore, a computer program product on a computer-readable storage medium (e.g., non-transitory) having processor-executable instructions (e.g., computer software) embodied in the storage medium. Any suitable computer-readable storage medium may be utilized including hard disks, CD-ROMs, optical storage devices, magnetic storage devices, memresistors, Non-Volatile Random Access Memory (NVRAM), flash memory, or a combination thereof.

Throughout this application reference is made to block diagrams and flowcharts. It will be understood that each block of the block diagrams and flowcharts, and combinations of blocks in the block diagrams and flowcharts, respectively, may be implemented by processor-executable instructions. These processor-executable instructions may be loaded onto a general purpose computer, special purpose computer, or other programmable data processing apparatus to produce a machine, such that the processor-executable instructions which execute on the computer or other programmable data processing apparatus create a device for implementing the functions specified in the flowchart block or blocks.

These processor-executable instructions may also be stored in a computer-readable memory that may direct a computer or other programmable data processing apparatus to function in a particular manner, such that the processor-executable instructions stored in the computer-readable memory produce an article of manufacture including processor-executable instructions for implementing the function specified in the flowchart block or blocks. The processor-executable instructions may also be loaded onto a computer or other programmable data processing apparatus to cause a series of operational steps to be performed on the computer or other programmable apparatus to produce a computer-implemented process such that the processor-executable instructions that execute on the computer or other programmable apparatus provide steps for implementing the functions specified in the flowchart block or blocks.

Blocks of the block diagrams and flowcharts support combinations of devices for performing the specified functions, combinations of steps for performing the specified functions and program instruction means for performing the specified functions. It will also be understood that each block of the block diagrams and flowcharts, and combinations of blocks in the block diagrams and flowcharts, may be implemented by special purpose hardware-based computer systems that perform the specified functions or steps, or combinations of special purpose hardware and computer instructions.

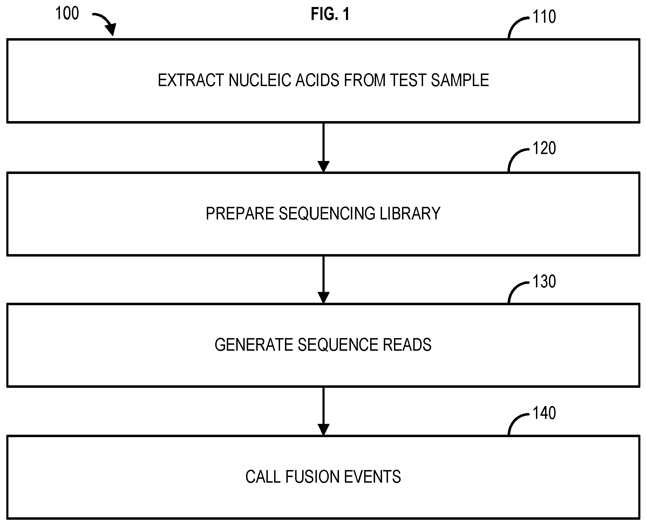

FIG. 1 is an example method 100 for processing a test sample obtained from an individual to call a fusion event. The test sample may be obtained from a patient. At step 110, nucleic acids (DNA or RNA) may be extracted from a test sample. In an embodiment, the nucleic acids comprise cell-free nucleic acids. In various embodiments, the test sample may be a sample selected from one or more of blood, plasma, serum, urine, fecal, saliva samples, combinations thereof, and/or the like. Alternatively, the biological sample may comprise a sample selected from one or more of whole blood, a blood fraction, a tissue biopsy, pleural fluid, pericardial fluid, cerebrospinal fluid, and peritoneal fluid. In one embodiment, the test sample may comprise cell-free nucleic acids, examples of which are cell-free DNA and/or cell-free RNA. For example, the test sample may be a cell-free nucleic acid sample taken from a subject's blood. In one embodiment, the cell free nucleic acid sample may be extracted from a test sample obtained from a subject known to have cancer (e.g., a cancer patient), or a subject suspected of having cancer.

The following description related to fusion calling may be applicable to both DNA and RNA types of nucleic acid sequences. In various embodiments, nucleic acids are extracted from the test sample through a purification process. In general, any known method in the art can be used for purifying nucleic acids. For example, nucleic acids can be isolated by pelleting and/or precipitating the nucleic acids in a tube. In some embodiments, nucleic acids can be further processed. For example, the cell free nucleic acid extracted from the test sample can be RNA that is then converted to DNA using reverse transcriptase.

In some aspects, the method 100 comprises step 110. In some aspects, the method 100 may begin at step 120 using nucleic acids obtained from a test sample.

The method 100 may comprise preparation of a sequencing library at step 120. During library preparation, adapters, for example, include one or more sequencing oligonucleotides for use in subsequent cluster generation and/or sequencing (e.g., known P5 and P7 sequences for used in sequencing by synthesis (SBS) (Illumina, San Diego, Calif.)) may be ligated to the ends of the nucleic acid molecules through adapter ligation. In one embodiment, molecular barcodes may be added to the extracted nucleic acids during adapter ligation. In some embodiments, molecular barcodes are degenerate base pairs that serve as a unique tag that can be used to identify sequence reads obtained from nucleic acids. In other embodiments, the molecular barcodes are selected from a limited set of molecular barcodes (e.g., 2 to 1,000,000; 2 to 100,000; 2 to 10,000; 2 to 1,000 different molecular barcode sequences). In some embodiments, the number of molecular barcodes in the set of molecular barcodes is less than the number of polynucleotides in a sample. In some embodiments with a limited number of molecular barcodes in a set, the molecular barcodes may comprise non-degenerate base pairs that can be used to distinguish different molecules based on sequence information from the molecular barcodes and genomic coordinate information based on where the sequence reads map on a reference sequence. In some embodiments, the molecular barcodes are short nucleic acid sequences (e.g., 4-10 base pairs) that are added to ends of nucleic acids during adapter ligation. The molecular barcodes can be further replicated along with the attached nucleic acids during amplification, which provides a way to identify sequence reads that originate from the same original nucleic acid segment in downstream analysis.

In an embodiment, step 120 may optionally comprise hybridizing nucleic acids using hybridization probes and/or performing enrichment on nucleic acid fragments. For example, when generating sequence reads through a targeted gene panel or when generating sequence reads through whole exome sequencing. Conversely, hybridizing nucleic acids using hybridization probes and/or performing enrichment on nucleic acid fragments are not performed when generating sequence reads through whole genome sequencing. Hybridizing nucleic acids using hybridization probes may comprise using hybridization probes to enrich a sequencing library for a selected set of nucleic acids. Hybridization probes can be designed to target and hybridize with targeted nucleic acid sequences to pull down and enrich targeted nucleic acid molecules that may be informative for the presence or absence of cancer (or disease), cancer status, or a cancer classification (e.g., cancer type or tissue of origin). In accordance with this step, a plurality of hybridization pull down probes can be used for a given target sequence or gene. The probes can range in length from about 40 to about 160 base pairs (bp), from about 60 to about 120 bp, or from about 70 bp to about 100 bp. In one embodiment, the probes cover overlapping portions of the target region or gene. For targeted gene panel sequencing, the hybridization probes may be designed to target and pull down nucleic acid molecules that derive from specific gene sequences that are included in the targeted gene panel. For whole exome sequencing, the hybridization probes may be designed to target and pull down nucleic acid molecules that derive from exon sequences in a reference genome. Subsequently, the hybridized nucleic acid molecules may be enriched. For example, the hybridized nucleic acid molecules can be captured and amplified using PCR. The target sequences can be enriched to obtain enriched sequences that can be subsequently sequenced. For example, as is well known in the art, a biotin moiety can be added to the 5'-end of the probes (i.e., biotinylated) to facilitate pulling down of target probe-nucleic acids complexes using a streptavidin-coated surface (e.g., streptavidin-coated beads). This may improve the sequencing depth of sequence reads. However, PCR is imperfect; it introduces artifacts (e.g., skews and new hybrid or erroneous sequences) into the pool of amplified DNA molecules. For example, template switching, a process by which two templates combine to form a novel chimeric product during amplification may produce artifacts. PCR template switching produces hybrid sequences of two sequences already present in the input. DNA polymerase can jump from one template to another in a region of complementarity without aborting the nascent DNA strand during PCR. This nascent strand therefore has a new hybrid sequence, where one piece is complementary to the old template and the other piece is complementary to the new template. Similarly, nascent transcripts can be aborted before completion and then might act as primers in a subsequent cycle of PCR, again resulting in a new hybrid species.

In some aspects, the method 100 comprises steps 110 and 120. In some aspects, the method 100 may begin at step 120 using nucleic acids obtained from a test sample. In some aspects, the method 100 may begin at step 130 using a previously prepared sequence library. In some aspects, a previously prepared sequence library can be purchased.

The method 100 may comprise sequencing the nucleic acids in the sequencing library to generate sequence reads at step 130. Sequence reads may be acquired by known means in the art. For example, a number of techniques and platforms obtain sequence reads directly from millions of individual nucleic acid (e.g., DNA such as cfDNA or gDNA or RNA such as cfRNA) molecules in parallel. Such techniques can be suitable for performing any of targeted gene panel sequencing, whole exome sequencing, whole genome sequencing, targeted gene panel bisulfite sequencing, and whole genome bisulfite sequencing.

As a first example, sequencing-by-synthesis technologies rely on the detection of fluorescent nucleotides as they are incorporated into a nascent strand of DNA that is complementary to the template being sequenced. In one method, oligonucleotides 30-50 bases in length are covalently anchored at the 5' end to glass cover slips. These anchored strands perform two functions. First, they act as capture sites for the target template strands if the templates are configured with capture tails complementary to the surface-bound oligonucleotides. They also act as primers for the template directed primer extension that forms the basis of the sequence reading. The capture primers function as a fixed position site for sequence determination using multiple cycles of synthesis, detection, and chemical cleavage of the dye-linker to remove the dye. Each cycle consists of adding the polymerase/labeled nucleotide mixture, rinsing, imaging and cleavage of dye.

In an alternative method, polymerase is modified with a fluorescent donor molecule and immobilized on a glass slide, while each nucleotide is color-coded with an acceptor fluorescent moiety attached to a gamma-phosphate. The system detects the interaction between a fluorescently-tagged polymerase and a fluorescently modified nucleotide as the nucleotide becomes incorporated into the *de novo* chain.

Any suitable sequencing-by-synthesis platform can be used to identify mutations. Sequencing-by-synthesis platforms include the Genome Sequencers from Roche/454 Life Sciences, the GENOME ANALYZER from Illumina/SOLEXA, the SOLID system from Applied BioSystems, and the HELISCOPE system from Helicos Biosciences. Sequencing-by-synthesis platforms have also been described by VisiGen Biotechnologies. In some embodiments, a plurality of nucleic acid molecules being sequenced is bound to a support (e.g., solid support). To immobilize the nucleic acid on a support, a capture sequence/universal priming site can be added at the 3' and/or 5' end of the template. The nucleic acids can be bound to the support by hybridizing the capture sequence to a complementary sequence covalently attached to the support. The capture sequence (also referred to as a universal capture sequence) is a nucleic acid sequence complementary to a sequence attached to a support that may dually serve as a universal primer.

As an alternative to a capture sequence, a member of a coupling pair (such as, e.g., antibody/antigen, receptor/ligand, or the avidin-biotin pair) can be linked to each molecule to be captured on a surface coated with a respective second member of that coupling pair. Subsequent to the capture, the sequence can be analyzed, for example, by single molecule detection/sequencing, including template-dependent sequencing-by-synthesis. In sequencing-by-synthesis, the surface-bound molecule is exposed to a plurality of labeled nucleotide triphosphates in the presence of polymerase. The sequence of the template is determined by the order of labeled nucleotides incorporated into the 3' end of the growing chain. This can be done in real time or can be done in a step-and-repeat mode. For real-time analysis, different optical labels to each nucleotide can be incorporated and multiple lasers can be utilized for stimulation of incorporated nucleotides.

Massively parallel sequencing or next generation sequencing (NGS) techniques include synthesis technology, pyrosequencing, ion semiconductor technology, single-molecule real-time sequencing, sequencing by ligation, or paired-end sequencing. Examples of massively parallel sequencing platforms are the Illumina HISEQ or MISEQ, ION PERSONAL GENOME MACHINE, the PACBIO RSII sequencer or SEQUEL System, Qiagen's GENEREADER, and the Oxford MINION. Additional similar current massively parallel sequencing technologies can be used, as well as future generations of these technologies.

In various embodiments, a sequence read may be comprised of a read pair denoted as R1 and R2. For example, the first read R1 may be sequenced from a first end of a nucleic acid molecule whereas the second read R2 may be sequenced from the second end of the nucleic acid molecule.

In an embodiment, at step 130, the sequence reads may undergo further processing. In an embodiment, rather than generating the sequence reads through steps 110-130, the sequence reads may be obtained, downloaded, determined, received, and the like, from any available data source. The sequence reads may be obtained, downloaded, determined, received, and the like, for example, from whole exome sequencing (WES) data (DNA-seq), whole genome sequencing (WGS) data (DNA-seq), and/or transcriptome sequencing (RNA-seq) data. The methods and systems described may obtain the sequence reads in one of a variety of formats (e.g., FASTA, FASTQ, and/or other proprietary format), depending, for example, on the sequencing platform that is used to generate the sequence reads. Thus, obtaining the sequence reads from a sequencing platform can include standardization of the read format in such a way that the sequence reads can be used for further processing and analysis described herein. One non-limiting example of standardizing sequence format is adjusting quality score format of the sequence reads. In some embodiments, the structure of a data file containing the sequence reads can be optimized to enhance (e.g., accelerated or more efficient) retrieval of the data file.

The further processing may include, for example, a pre-filtering step to remove sequence reads, stitching read pairs, and/or overhang trimming of read pairs. Pre-filtering may comprise removing sequence reads that meet one or more criteria. Examples of the criteria include, but are not limited to: identifying whether a sequence read is a singleton, identifying whether a sequence read is a hard clip, filtering based on a template length (TLEN) (e.g., a threshold TLEN), filtering based on an alignment score (e.g., a threshold alignment score), or filtering based on a base quality score (e.g., a threshold of a median or mean base quality score). Another criterion includes determining that if a sequence read pair meets the criterion that the reads of the read pair are from differing chromosomes, then the sequence read pair is maintained and not filtered out. Additional examples of criteria include filtering based on a bit flag, a cigar, an edit distance (e.g., a minimum or maximum edit distance), a suboptimal alignment score, or a supplementary alignment measure.

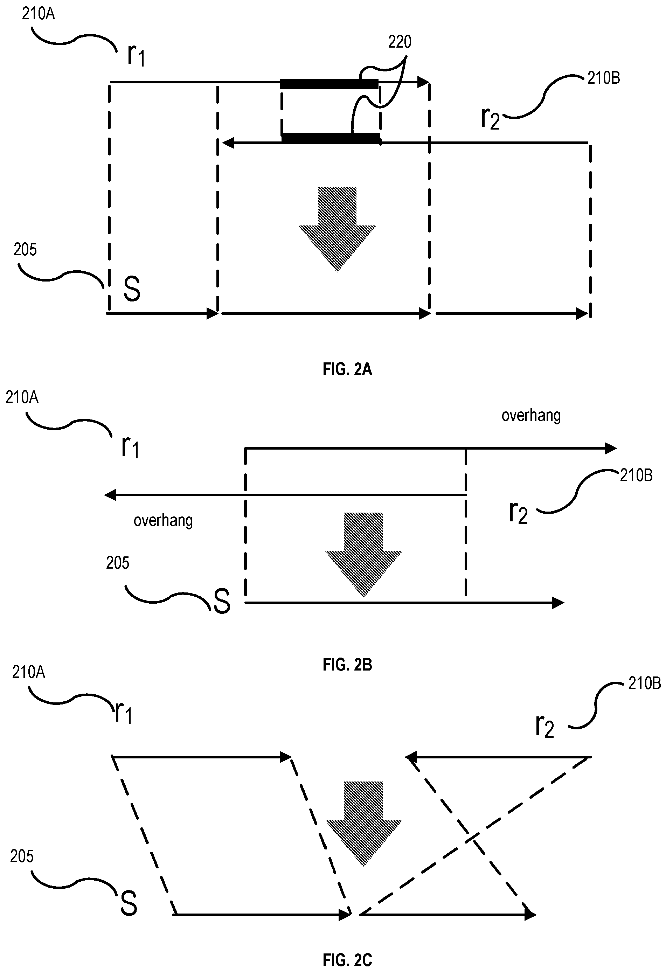

FIG. 2A, FIG. 2B, and FIG. 2C depict example stitching and trimming processes for generating a fragment s 205 from a read pair r₁ 210 A and r₂ 210 B, in accordance with an embodiment.

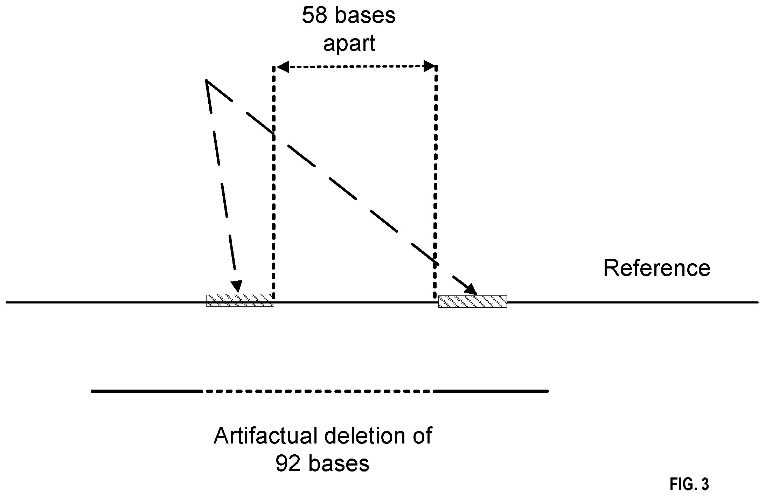

As shown in FIG. 2A, FIG. 2B, and FIG. 2C, r₁ 210 A and r₂ 210 B are represented as arrows facing each other denoting the forward and reverse complement strands. The read pair (r₁, r₂) are evaluated to determine whether they should be stitched into the same fragment s 205: r₁ and r₂ are decomposed to kmers, and each common kmer anchors the suffix-prefix alignment of r₁ 210 A and r₂ 210 B (FIG. 2A). If the similarity of the alignment passes a certain threshold, stitching is applied. As shown in FIG. 2A, the overlapping regions 220 between the read pair denotes one of the shared kmers (e.g., overlap) between them, which is an anchor for suffix-prefix alignment. Therefore, the stitched fragment s 205 is a concatenation of a prefix of r₁ 210 A, overlap, and a suffix of r₂ 210 B. At times, the stitching code fuses long molecules at the perfect repeat, and this causes an artifact resembling a fusion. Read mates are stitched *de novo,* but neighboring perfect repeats may cause long molecules to be stitched incorrectly, as shown in the FIG. 3.

In another scenario, if the 3' end of r₁/r₂ extends beyond the 5' of r₂/r₁ (overhang), fragment s 205 becomes the overlapping region. This is the scenario shown in FIG. 2B where r₁ 210 A and/or r₂ 210 B extends beyond the 5' region of the other read. The overhang is trimmed, and fragment s 205 is the overlap.

In another scenario, as shown in FIG. 2C, if r₁ 210 A and r₂ 210 B cannot be stitched, either because they are not overlapping and/or there are too many sequencing errors, the paired reads are concatenated to form fragment s 205, where reverse complementing r₂ 210 B converts both read into the same strand. A non-alphabetical character that would not be contained in any kmer is arbitrarily chosen to prevent the generation of non-existent kmers from the data.

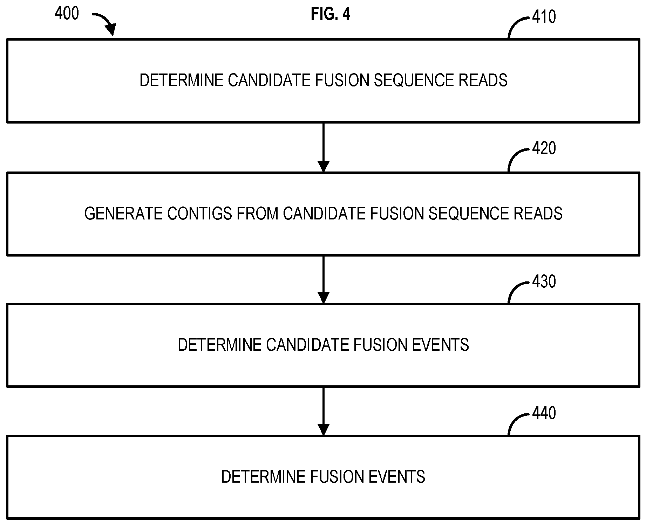

The method 100 may comprise processing the sequence reads using a computational analysis to call a fusion event at step 140. Such a computational analysis is now described in relation to FIG. 4, which depicts a method 400 of identifying fusion events, in accordance with an embodiment. Generally, the computational analysis is an *de novo* fusion caller that is configured to predict the presence of a fusion event(s) in the individual without prior knowledge.

The method 400 may comprise determining candidate fusion sequence reads at step 410, generating contigs from candidate fusion sequence reads at step 420, determining candidate fusion events at step 430, and determining fusion events at step 440.

Determining candidate fusion sequence reads at step 410 may comprise aligning a plurality of sequence reads to a reference sequence. The reference sequence may comprise DNA sequences across a region of the genome, such as a chromosome. The reference sequence including DNA sequences across the region of the genome can be used to identify candidate fusion events that affect that particular region of the genome. The reference sequence may comprise exonic DNA sequences. Thus, the reference sequence can be used to identify candidate fusion events that affect exonic DNA sequences. In some embodiments, the reference sequence may comprise, in addition to exonic DNA sequences, intronic DNA sequences. Thus, the reference sequence may be used to identify candidate fusion events that affect both exonic and intronic DNA sequences. In some embodiments, the reference sequence may comprise a combination of exonic DNA sequences, intronic DNA sequences, and additional nucleotide bases within padding regions. Padding regions can be nucleic acid sequences that are known to be unlikely associated with gene fusion events such as repeating nucleic acid sequences or other intronic regions. Thus, the reference sequence may be used to identify candidate fusion events that affect exonic DNA sequences, intronic DNA sequences, as well as junctions between exonic/intronic DNA sequences.

Alignment of the plurality of sequence reads to the reference sequence may comprise any alignment technique as known in the art. Examples of alignment techniques include, but are not limited to, pairwise alignment and multiple sequence alignment. Pairwise alignment may comprise, for example, exhaustive or heuristic (e.g., not exhaustive) pairwise alignment. Exhaustive pairwise alignment, sometimes called a "brute force" approach, calculates an alignment score for every possible alignment between every possible pair of sequences among a set. Multiple sequence alignment may comprise progressive alignment, as implemented by the program ClustalW (see, e.g., Thompson, et al., Nucl. Acids. Res., 22:4673-80 (1994)). A result of the alignment may comprise one or more Binary Alignment Map (BAM) files.

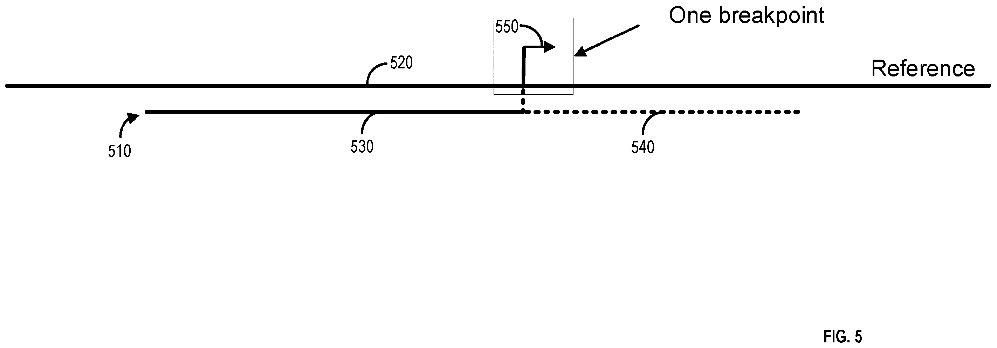

Determining candidate fusion sequence reads at step 410 may further comprise determining one or more breakpoints in an alignment of at least one sequence read of the plurality of sequence reads to the reference sequence. Any sequence reads associated with the one or more breakpoints in the alignment may be identified as candidate fusion sequence reads. A breakpoint may be a region or point where the sequence read has altered from the reference sequence. The alignments of each sequence read may contribute one or more breakpoints. A breakpoint may be an oriented position on a chromosome. Presence of breakpoints in the alignment may indicate either an error in the sequencing process or a genuine signal for a true fusion events. FIG. 5 shows an example of a sequence read 510 that is determined to be a candidate fusion sequence read. The sequence read 510 is aligned to a reference sequence 520. A first potion 530 of the sequence read 510 is well aligned to the reference sequence 520 however, a second portion 540 is not well aligned to the reference sequence 520 starting at a breakpoint 550. The sequence read 510 may be considered a candidate fusion sequence read based on the presence of the breakpoint 550. While not shown in FIG. 5, another breakpoint will be generated from the other alignment for the same sequence read 510.

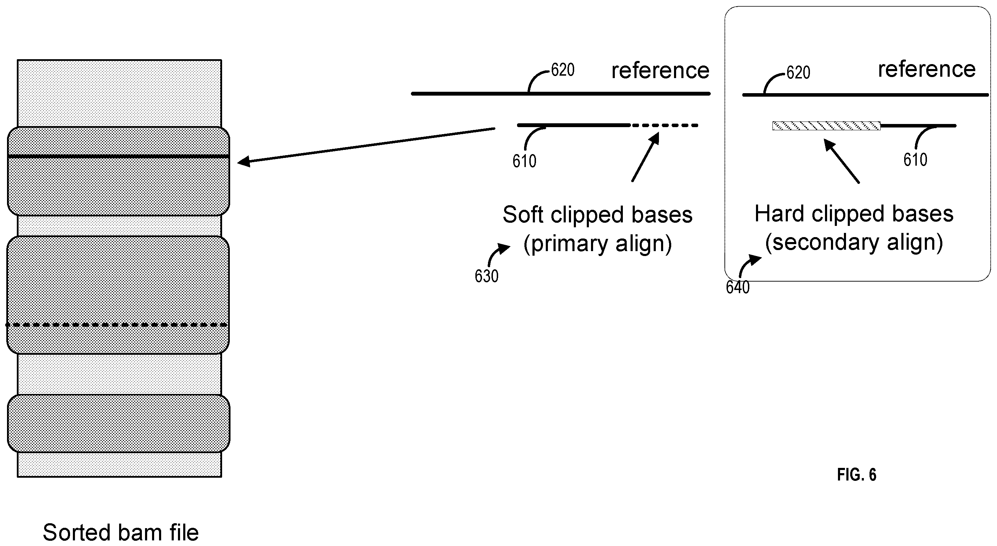

In an embodiment, one or more BAM files may be queried to determine sequence reads that should be discarded and/or considered as candidate fusion sequence reads. The BAM files may be scanned and any logical sequence reads may be discarded. Logical sequence reads may comprise reads that do not appear to contain a fusion event (e.g., no hard-clipping, no soft-clipping). In an embodiment, a minimum alignment length and/or a maximum alignment length may be used to identify logical sequence reads. The minimum alignment length may be, for example, from and including 1-100. In an embodiment, the minimum alignment length may be 40. The maximum alignment length may be, for example, from and including 600-1000. In an embodiment, the maximum alignment length may be 800. Any sequence reads that contain a number of bases aligned to a reference sequence below the minimum alignment length or above the maximum alignment length are not considered to be logical sequence reads and may be retained for further analysis. In an embodiment, sequence reads associated with low mapping quality scores (MAPQ) may be discarded. A low mapping quality score may be for example, anywhere from, and including, 0 to 60. In an embodiment, a low mapping quality score may be 50 or less. Sequence reads comprising indels larger than a threshold may be retained as candidate fusion sequence read. The threshold may be for example, anywhere from, and including, 15 to 30 bases. In an embodiment, the threshold may be 24 bases. FIG. 6 shows an example of a sequence read 610 that is determined to be a candidate fusion sequence read. The sequence read 610 has two alignments to a reference sequence 620. A primary alignment 630 wherein portions of the sequence read 610 do not match well to the reference sequence 620 on either side of the sequence read 610 (soft clipped bases) and a secondary alignment 640 wherein the sequence read 610 could align reasonably well to more than one place in the reference sequence 620 and includes a portion of the sequence read 610 that has been removed prior to alignment (hard clipped bases).

Returning to FIG. 4, generating contigs from candidate fusion sequence reads at step 420 may comprise grouping the candidate fusion sequence reads into groups (or "containers" or "packets") based on one or more common breakpoints and assembling the candidate fusion sequence reads in each packet into one or more contigs. The candidate fusion sequence reads sharing the same or neighboring breakpoints (e.g., common breakpoints) may be placed into the same packet/container. In an embodiment, a common breakpoint may be: 1) a breakpoint on each of two candidate fusion sequence reads that are in the same chromosome with the same orientation and/or 2) a breakpoint on each of two candidate fusion sequence reads at the same position or within a threshold number of bases (e.g., within a threshold of anywhere from, and including, 1 to 40 bases, for example 12 bases) and with the same orientation. In another embodiment, a compatibility test for two vectors of breakpoints may be performed.

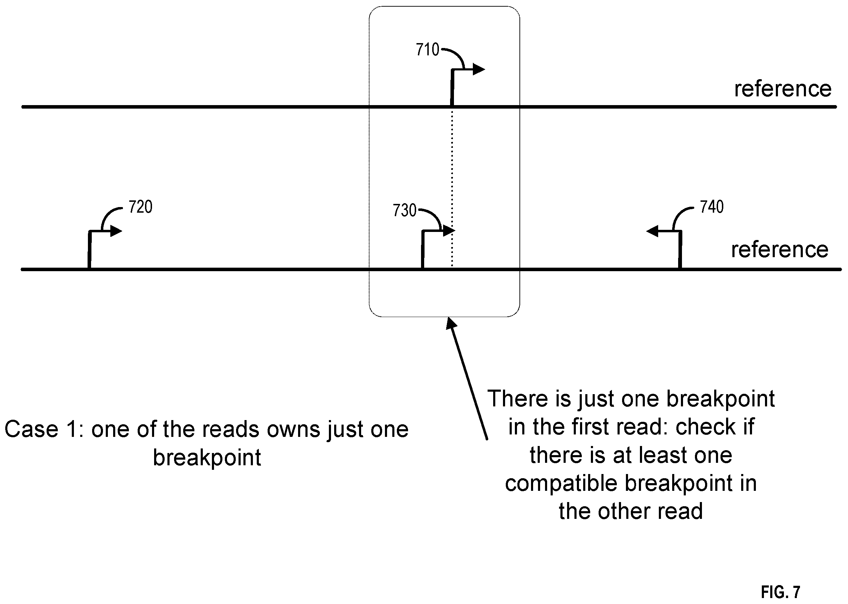

FIG. 7 shows a scenario where a candidate fusion sequence read comprises a single breakpoint and another candidate fusion sequence read comprises multiple breakpoints. A first candidate fusion sequence read comprises a breakpoint 710 and a second candidate fusion sequence read comprises a breakpoint 720, a breakpoint 730, and a breakpoint 740. The breakpoint 720 and the breakpoint 740 are not at positions within a threshold number of bases from the position of breakpoint 710, and therefore do not contribute to grouping the first candidate fusion sequence read and the second candidate fusion sequence read. However, the positions of the breakpoint 710 and the breakpoint 730 are within the threshold number of bases and may serve as a basis for grouping the first candidate fusion sequence read and the second candidate fusion sequence read into the same packet.

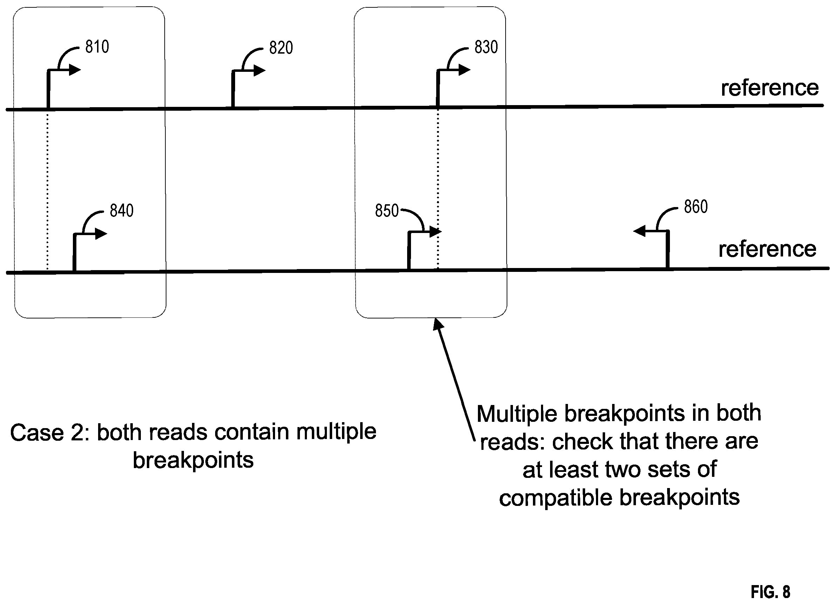

FIG. 8 shows a scenario where a candidate fusion sequence read comprises multiple breakpoints and another candidate fusion sequence read also comprises multiple breakpoints. A first candidate fusion sequence read comprises a breakpoint 810, a breakpoint 820, and a breakpoint 830. A second candidate fusion sequence read comprises a breakpoint 840, a breakpoint 850, and a breakpoint 860. A comparison may be made for each breakpoint of the first candidate fusion sequence read to each breakpoint of the second candidate fusion sequence read. As shown in FIG. 8, the breakpoint 810 and the breakpoint 840 are at positions within a threshold number of bases and the breakpoint 830 and the breakpoint 860 are at positions within the threshold number of bases. These pairs of breakpoints may serve as a basis for grouping the first candidate fusion sequence read and the second candidate fusion sequence read into the same packet. However, the breakpoint 820 and the breakpoint 860 are not within the threshold number of bases of any other breakpoint, and therefore do not contribute to grouping the first candidate fusion sequence read and the second candidate fusion sequence read.

In an embodiment, a packet of candidate fusion sequence reads may be computationally generated by constructing one or more container data structures. In an embodiment, the one or more container data structures may comprise one or more graph data structures. The graph data structure may comprise nodes representing candidate fusion sequence reads and edges connecting the nodes representing compatible candidate fusion sequence reads. Each connected node may be considered part of a packet. Graph data structure construction may be parallelized given the computationally intensive nature of such construction.

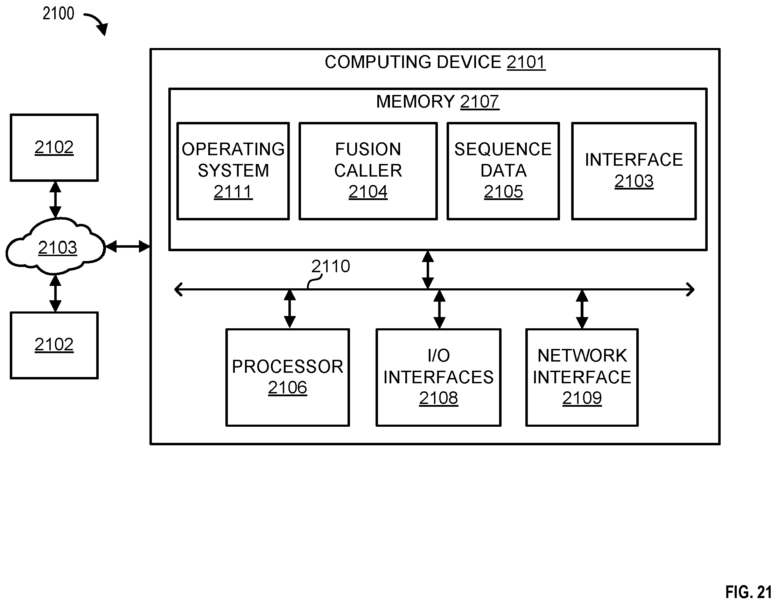

The graph data structure may comprise a type of data structure in which pairs of vertices (also referred to as nodes) are connected by edges. In an embodiment, the graph data structure is stored in a memory subsystem (e.g., FIG. 21, memory 2107), which may include pointers to identify a physical location in the memory 2107 where each vertex is stored. Typically, the nodes in a graph data structure each represent an element in a set, while the edges represent relationships among the elements. The graph data structure may comprise a directed graph, a tree, a directed acyclic graph (DAG), and/or the like. A directed graph is one in which the edges have a direction. A tree is a type of directed graph data structure having a root node, and a number of additional nodes that are each either an internal node or a leaf node. The root node and internal nodes each have one or more "child" nodes and each is referred to as the "parent" of its child nodes. Leaf nodes do not have any child nodes. Edges in a tree are conventionally directed from parent to child. In a tree, nodes have exactly one parent. A generalization of trees, known as a directed acyclic graph (DAG), allows a node to have multiple parents, but does not allow the edges to form a cycle.

In an embodiment, the graph data structure may represent a de Bruijn graph. De Bruijn graphs reduce the computation effort by breaking reads into smaller sequences of DNA, called *k*-mers, where the parameter *k* denotes the length in bases of these sequences. In a de Bruijn graph, all reads are broken into *k*-mers (all subsequences of length *k* within the reads) and a path between the *k*-mers is calculated. In assembly according to this method, the reads are represented as a path through the *k*-mers. The de Bruijn graph captures overlaps of length *k*-1 between these *k*-mers and not between the actual reads. Thus, for example, the sequence CATGGA could be represented as a path through the following 2-mers: CA, AT, TG, GG, and GA. Other *k*-mers are contemplated, for example, 1-mer, 3-mer, 4-mer, 5-mer, 6-mer, 7-mer, 8-mer, etc. The de Bruijn graph approach handles redundancy well and makes the computation of complex paths tractable. By reducing the entire data set down to *k*-mer overlaps, the de Bruijn graph reduces the high redundancy in short-read data sets. The maximum efficient *k*-mer size for a particular assembly may be determined by the read length as well as the error rate. The value of the parameter *k* has significant influence on the quality of the assembly. Estimates of good values can be made before the assembly, or the optimal value can be found by testing a small range of values.

In an embodiment, each of the candidate fusion sequence reads may comprise a string of symbols. For example, string s may be a sequence of symbols drawn from an alphabet **A.** The length of *s* is denoted by |*s*|. A substring of s is a string occurring in *s*: it has a starting position *i* and a length *l* and is denoted by *s*(*i,l*). A substring of length *l* is also denoted an *l*-mer. In the following, assume **A** is the DNA alphabet **A** ={A,C,G,T} for which symbols have complements: (A,T) and (C,G) are the complementing pairs. The reverse-complemented string *s̅* is the reverse sequence of complemented symbols in s. The canonical string *ŝ* is the lexicographically smallest of *s* and its reverse-complement *S̅*. The minimizer of an *l*-mer *x* is a *g*-mer *y* occurring in *x* such that *g*<1 and *y* is the lexicographically smallest of all the *g*-mers in *x*. The lexicographical order can be cumbersome to use since poly-A *g*-mers naturally occur in sequencing data and is often replaced by a random order. The simplest way to obtain a random order is to compute a hash-value for each g-mer in x and select the g-mer with the smallest hash-value as the minimizer. In an embodiment, minimizers generated by random orderings may be used.

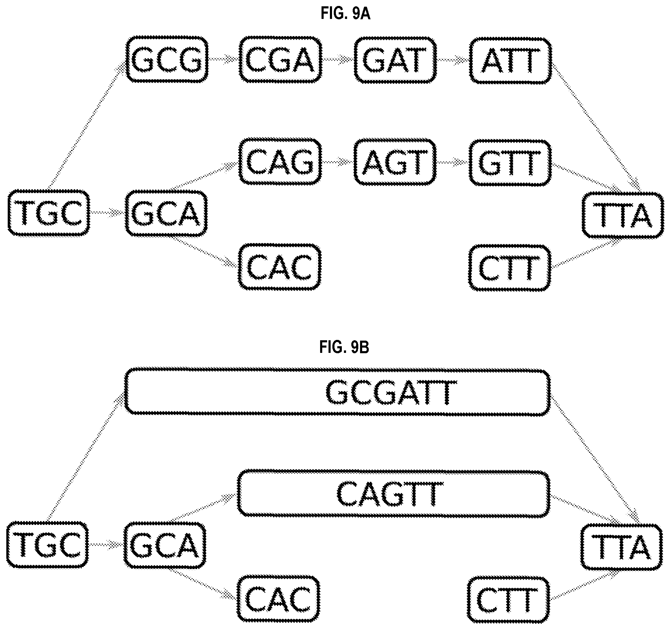

A de Bruijn graph (dBG) may be a directed graph *G*=(*V,E*) in which each vertex *v*∈*V* represents a *k*-mer. A directed edge *e*∈*E* from vertex v to vertex *v'* representing *k-*mers x and *x*', respectively, exists if and only if *x*(2,*k*-1)=*x*'(1,*k*-1). Each *k*-mer x has |**A**| possible successors *x*(2,*k*-1)⊙*a* and |**A**| possible predecessors *a*⊙*x*(1,*k*-1) in *G* with *a*∈**A** and ⊙ as the concatenation operator. Note that in the original combinatorial definition of the dBG, all possible *k*-mers for an alphabet **A** are present in the graph, whereas in the present embodiment, the definition is restricted to a subset of the de Bruijn graph representing the *k*-mers in the input. A path in the graph is a sequence of distinct and connected vertices *p*=(*v*₁,...,*vₘ*). The path *p* is non-branching if all its vertices have an in- and out-degree of one with exception of the head vertex *v*₁ which can have more than one incoming edge and the tail vertex *vₘ* which can have more than one outgoing edge. A non-branching path is maximal if it cannot be extended in the graph without being branching. A compacted de Bruijn graph (cdBG) merges all maximal non-branching paths of η vertices from the dBG into single vertices, called unitigs, representing words of length *k*+η-1. Minimal examples of dBG and cdBG are provided in FIG. 9A and FIG. 9B, respectively. Conventional techniques for generating the graph data structure include Bloom filters. However, Bloom filter data structures trade off memory usage and time complexity with a decreased false positive rate and poor data locality as bits corresponding to one element are scattered over a bitmap, resulting in several CPU cache misses when inserting and querying. To overcome these technical limitations, in an embodiment, a rolling hash function may be used to select a *g*-mer as the minimizer within a single *k*-mer. Since overlapping *k*-mers may share minimizers, an ascending minima approach may be used to recompute minimizers with amortized O(1) costs, so that iterating over minimizers of adjacent *k*-mers in a sequence is linear in the length of the sequence. Another optimization that may be implemented is to restrict the computation of minimizers to a subset of g-mers of a *k*-mer, namely, exclude the first and last *g*-mer as a candidate for being a minimizer. This ensures that for a given *k*-mer, all of its forward, respectively backward, adjacent *k*-mers necessarily share the same minimizer. While it is likely that a *k*-mer *x* and its neighbor *x*' share a minimizer, this neighbor hashing approach guarantees that when searching all forward, respectively backward, neighbors of *x*, they will all have the same minimizer and will be stored within the same block, thus minimizing cache misses.

In an embodiment, the graph data structure (e.g., representing a dBG or a cdBG) is stored in a memory subsystem (e.g., FIG. 21, memory 2107) using adjacency techniques, which may include pointers to identify a physical location in the memory 2107 where each vertex is stored. In an embodiment, the graph data structure is stored in the memory 2107 using adjacency lists. In some embodiments, there is an adjacency list for each vertex.

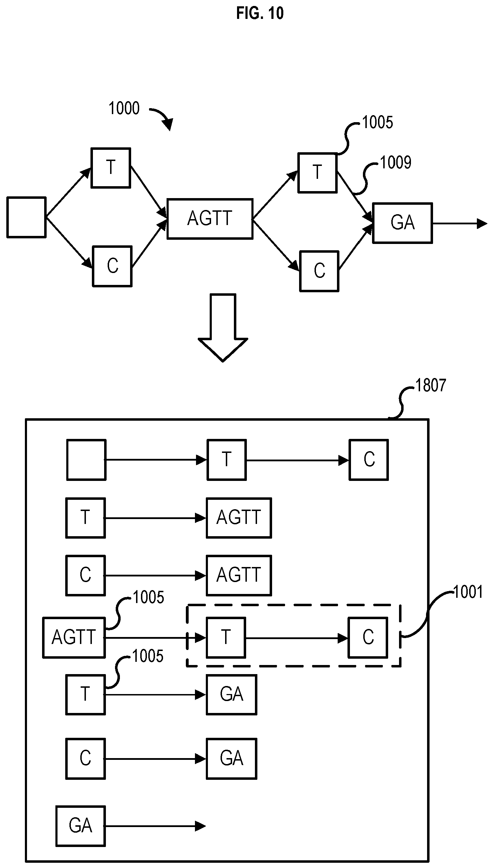

FIG. 10 shows a graph data structure 1000 that includes vertex objects 1005 and edge objects 1009. Portions of sequences (e.g., *k*-mers) are identified as blocks and those blocks are transformed into objects 1005 that are stored in a tangible memory device. It is noted that this object could potentially be stored using one byte of information. For example, if A=00, C=01, G=10, and T=11, then a block representing the string "AGTT" contains 00101111 (one byte). The objects 1005 are connected to create paths such that there is a path for each of the candidate fusion sequences. The paths are directed in the sense that the direction of each path corresponds to the 5' to 3' directionality of the nucleic acid. However, it is noted that it may be convenient or desirable to represent the sequence in a 3' to 5' direction and that doing so does not leave the scope of the invention. The connections creating the paths can themselves be implemented as objects so that the blocks are represented by vertex objects 1005 and the connections are represented by edge objects 1009. Thus the directed graph comprises vertex and edge objects stored in the tangible memory device. The graph data structure 1000 may represent a plurality of candidate fusion sequences in that each one of the original candidate fusion sequences can be retrieved by reading a path in the direction of that path. However, the graph data structure 1000 is a different article that the original candidate fusion sequences, at least in that portions of the sequences that match each other when aligned, have been transformed into single objects. The candidate fusion sequence strings may be stored within either the vertex objects 1005 or the edge objects 1009 (node and vertex are used synonymously). As used herein, node object 1005 and edge object 1009 refer to an object created using a computer system.

FIG. 10 further shows the use of an adjacency list 1001 for each vertex 1005. The disclosed methods and systems may use a processor to create a graph data structure **1000** that includes vertex objects 1005 and edge objects 1009 through the use of adjacency, e.g., adjacency lists or index free adjacency. Thus, the processor may create the graph data structure 1000 using index-free adjacency wherein a vertex 1005 includes a pointer to another vertex 1005 to which it is connected and the pointer identifies a physical location on a memory device 1807 where the connected vertex is stored. The graph data structure 1000 may be implemented using adjacency lists such that each vertex or edge stores a list of such objects that it is adjacent to. Each adjacency list comprises pointers to specific physical locations within a memory device for the adjacent objects.

The graph data structure 1000 will typically be stored on a physical device of memory subsystem 1807 in a fashion that provides for very rapid traversals. In that sense, the bottom portion of FIG. 10 represents that objects are stored at specific physical locations on a tangible part of the memory subsystem 1807. Each node 1005 is stored at a physical location, the location of which is referenced by a pointer in any adjacency list 1001 that references that node. Each node 1005 has an adjacency list 1001 that includes every adjacent node in the graph data structure 1000. The entries in the list 1001 are pointers to the adjacent nodes.

In certain embodiments, there is an adjacency list for each vertex and edge and the adjacency list for a vertex or edge lists the edges or vertices to which that vertex or edge is adjacent.

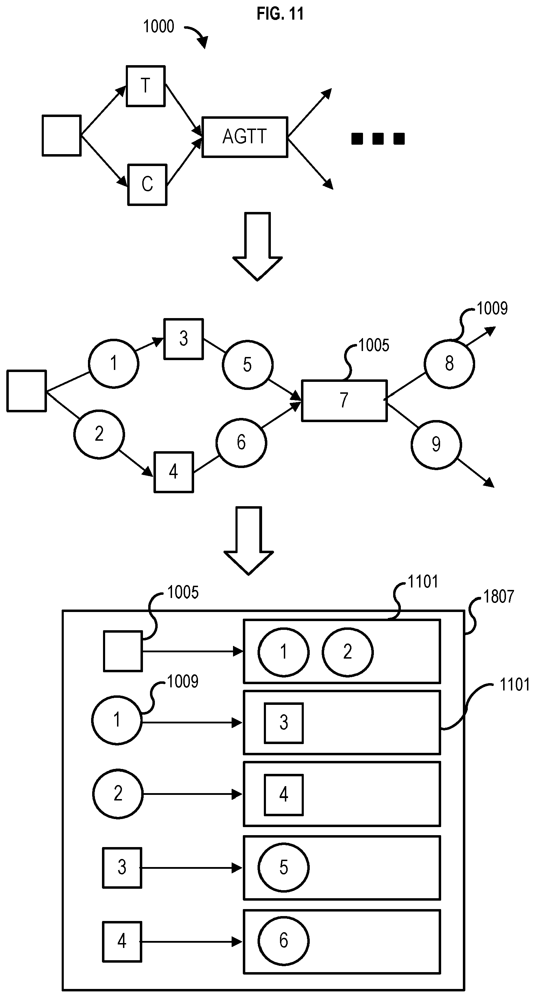

FIG. 11 shows the use of an adjacency list 1101 for each vertex 1005 and edge 1009. As shown in FIG. 11, the disclosed methods and systems may create the graph data structure 1000 using an adjacency list 1001 for each vertex and edge, wherein the adjacency list 1001 for a vertex 1005 or edge 1009 lists the edges or vertices to which that vertex or edge is adjacent. Each entry in adjacency list 1101 is a pointer to the adjacent vertex or edge.

Each pointer identifies a physical location in the memory subsystem at which the adjacent object is stored. In the preferred embodiments, the pointer or native pointer is manipulatable as a memory address in that it points to a physical location on the memory and permits access to the intended data by means of pointer dereference. That is, a pointer is a reference to a datum stored somewhere in memory; to obtain that datum is to dereference the pointer. The feature that separates pointers from other kinds of reference is that a pointer's value is interpreted as a memory address, at a low-level or hardware level. Such a graph representation provides means for fast random access, modification, and data retrieval.

In some embodiments, fast random access is supported and graph object storage are implemented with index-free adjacency in that every element contains a direct pointer to its adjacent elements, which obviates the need for index look-ups, allowing traversals to be very rapid. Index-free adjacency is another example of low-level, or hardware-level, memory referencing for data retrieval. Specifically, index-free adjacency can be implemented such that the pointers contained within elements are references to a physical location in memory.

Since a technological implementation that uses physical memory addressing such as native pointers can access and use data in such a lightweight fashion without the requirement of separate index tables or other intervening lookup steps, the capabilities of a given computer, e.g., any modern consumer-grade desktop computer, are extended to allow for full operation of a genomic-scale graph (e.g., a container data structure such as the graph data structure 1000 that represents a group of candidate fusion sequences). Thus storing graph elements (e.g., nodes and edges) using a library of objects with native pointers or other implementation that provides index-free adjacency actually improves the ability of the technology to provide storage, retrieval, and alignment for genomic information since it uses the physical memory of a computer in a particular way.

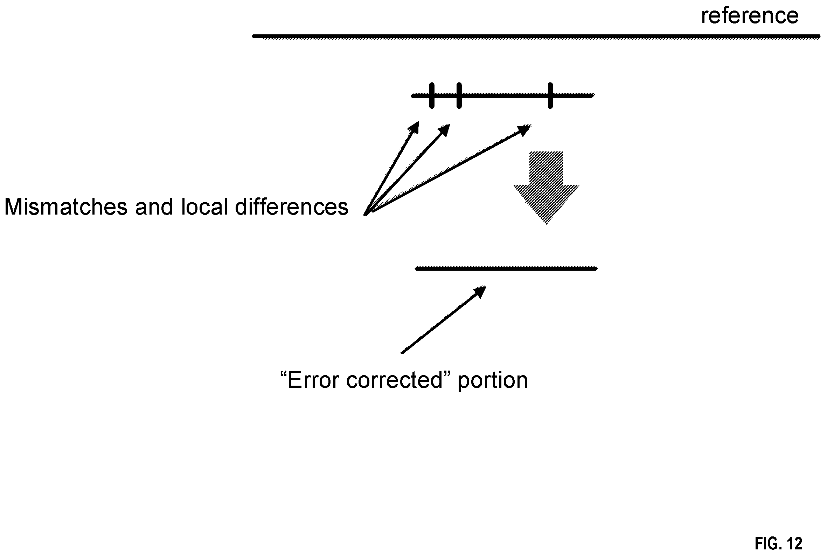

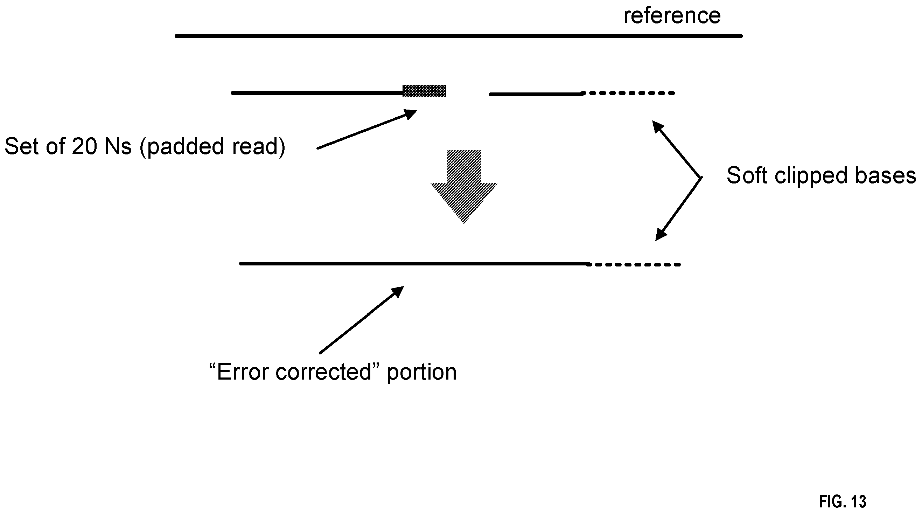

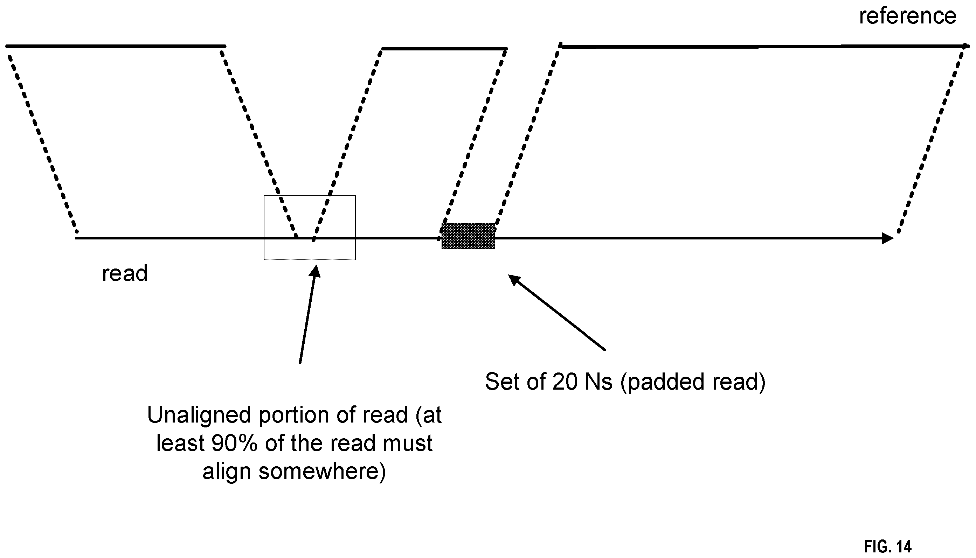

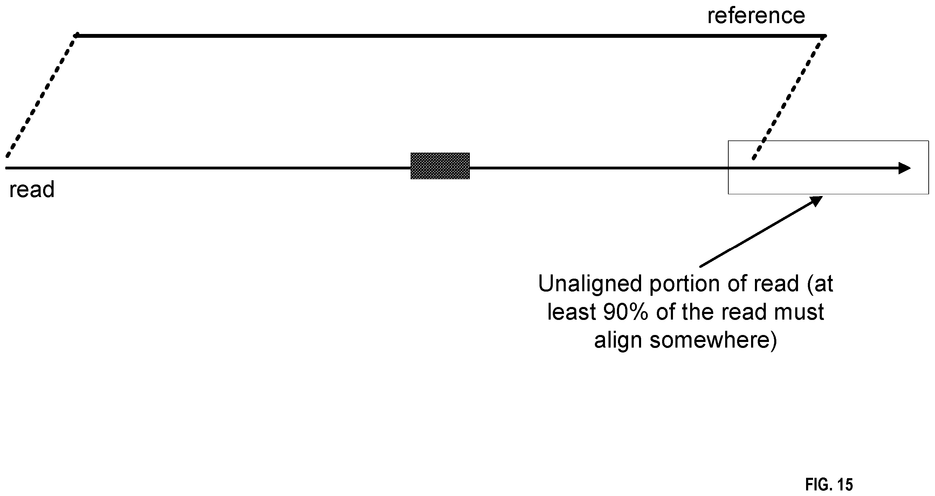

In an embodiment, an error correction procedure may be performed on the candidate fusion sequence reads in a given packet/container. The error correction procedure is designed to reduce the likelihood that a non-fusion event is identified as a fusion event. In an embodiment, indels greater than or equal to a threshold number of bases may be exempt from the error correction procedures. The threshold number of bases may be anywhere from, and including, 20 to 30 bases. In an embodiment, the threshold number of bases may be 24 bases. FIG. 12 shows an error correction procedure by which mismatches or local differences (e.g., variants) are replaced with corresponding bases from a reference sequence. FIG. 13 shows an error correction procedure applied to two candidate fusion sequence reads that align to a reference sequence within a threshold number of bases. One candidate fusion sequence read comprises a number of padding bases. The gap between the two candidate fusion sequence reads may be filled in using bases from the reference sequence at the same position as the gap. In an embodiment, the padding bases may be retained or may be replaced with bases from the reference sequence at the same position as the padding bases. A number of padding bases may be inserted between the two candidate fusion sequence reads, joining the two candidate fusion sequence reads as a single read. FIG. 14 shows an error correction procedure that discards candidate fusion sequence reads having an unaligned portion that exceed a threshold. For example, any candidate fusion sequence reads having an unaligned portion that is greater than or equal a threshold percentage of the candidate fusion sequence reads may be excluded. In an embodiment, the threshold percentage may be anywhere from, and including, 1% to 99%. In an embodiment, the threshold percentage may be 10%, meaning that any candidate fusion sequence reads having 10% or greater unaligned bases may be discarded. A practical result may be the exclusion of candidate fusion sequence reads comprising soft clipped bases. FIG. 15 further illustrates the error correction procedure of FIG. 14, whereby a candidate fusion sequence read having an unaligned portion that exceeds a threshold is excluded.

Assembling the remaining candidate fusion sequence reads in each packet/container into one or more contigs may comprise any known contig assembly method. For example, assembly by alignment can proceed by aligning sequence reads to each other or by aligning the sequence reads to a reference. For example, by aligning each read, in turn, to a reference genome, all of the reads are positioned in relationship to each other to create the assembly. In an embodiment, the container data structure for each packet may comprise a graph data structure representing a de Bruijn graph and assembling the candidate fusion sequence reads of each packet into contigs involves linearizing the de Bruijn graph to output the contig for each packet. For example, a greedy algorithm may be used to select edges of a de Bruijn graph that are most represented by sequence reads.

Returning to FIG. 4, determining candidate fusion events at step 430 may comprise aligning the contigs from each packet to the reference sequence and determining, based on the alignments, one or more candidate fusion events. As set out in the claims, a contig from a packet is aligned to a reference sequence (with decoys) and candidate fusion sequence reads for the packet are aligned to the contig. The candidate fusion sequence reads for the packet are clustered into families. A family includes candidate fusion sequence reads associated with the same molecule. A family may be determined based on molecular barcoding. Candidate fusion sequence reads containing the same molecular barcode may be grouped into the same family. In an embodiment, sequence reads containing the same molecular barcode and whose alignments begin within a number of bases (e.g., 30-50 bases) of each other may be grouped into the same family. One or more tests are applied to the resulting alignments to determine candidate fusion events. The one or more tests comprise a spread test and may further comprise a footprint test. The footprint test may comprise determining that a threshold number of families of candidate fusion sequence reads that support the contig span the breakpoint(s). The threshold may be for example, anywhere from, and including, 2 to 5 families. In an embodiment, the threshold may be 2 families. In an embodiment, the threshold may be 3 families The spread test comprises determining that a threshold amount of spread exists between sequence reads of at least two families of candidate fusion sequence reads that support the contig and span the breakpoint(s). The spread test involves aligning each sequence read to the contig. Then, for each sequence read, the start and stop coordinates, on the contig, for the first and last base are computed. The mean and standard deviation of all of the start points for each sequence read are calculated creating a mean start point and a start standard deviation. The mean and standard deviation of all of the stop points for each sequence read are calculated creating a mean stop point and a stop standard deviation. The spread is then defined as the minimum, or lowest, standard deviation between the start standard deviation and the stop standard deviation. Thus, in some embodiments, it is understood that only standard deviations are used to define the spread test. The threshold for the spread test may be from, and including, 1-15 bases. In an embodiment, the threshold may be 8 bases. If the spread is less than 8, then the fusion fails the spread test and it is discarded. In an embodiment, the threshold may be 7 bases. In an embodiment, the threshold may be 6 bases. In an embodiment, the threshold may be 5 bases.

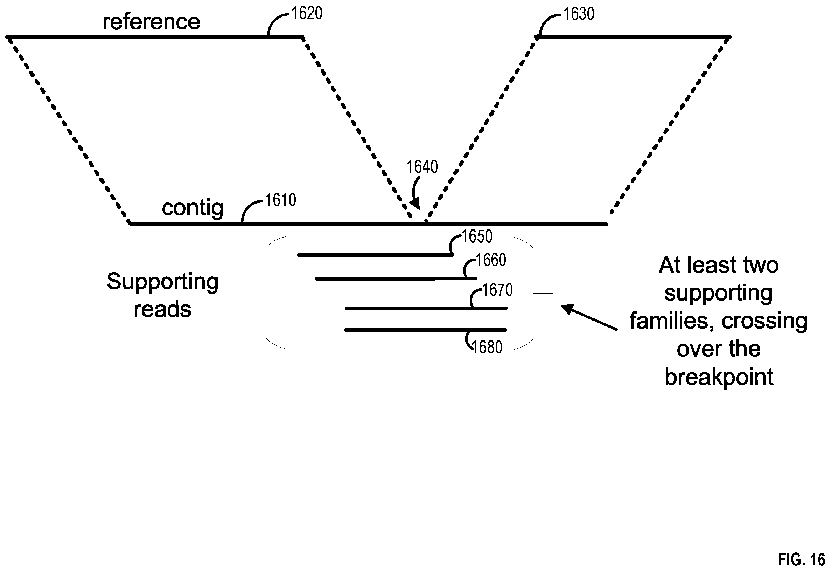

The footprint test is shown in FIG. 16. FIG. 16 shows a contig 1610 aligned to a first portion of a reference sequence 1620 and a second portion of the reference sequence 1630. A breakpoint 1640 exists between the aligned portions. The candidate fusion sequence reads that support the contig are indicated as a candidate fusion sequence read 1650, a candidate fusion sequence read 1660, a candidate fusion sequence read 1670, and a candidate fusion sequence read 1680. The candidate fusion sequence read 1650 belongs to a first family, the candidate fusion sequence read 1660 belongs to a second family, and the candidate fusion sequence read 1670 and the candidate fusion sequence read 1680 belong to a third family. As shown in FIG. 16, at least two families of candidate fusion sequence reads that support the contig span the breakpoint 1640, resulting in identification of the breakpoint 1640 as a candidate fusion event.

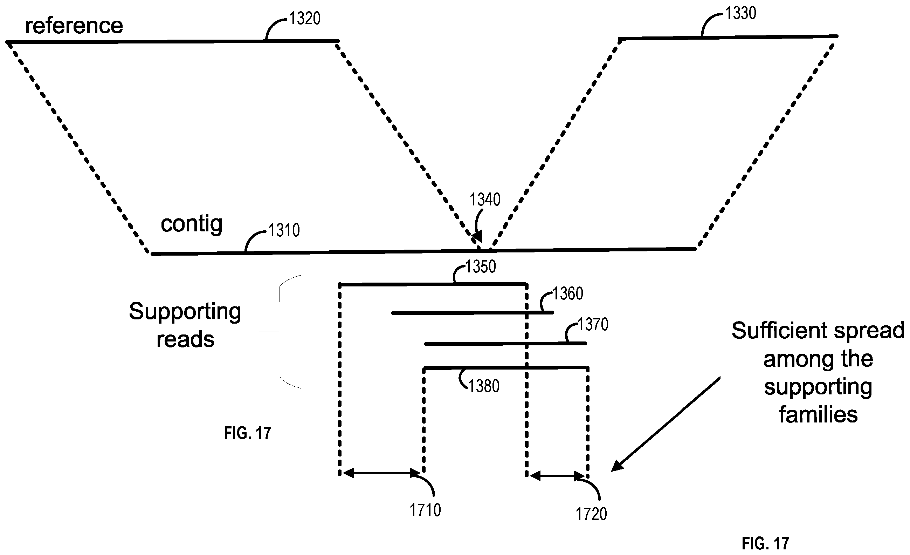

The spread test is shown in FIG. 17. As shown, for each sequence read 1650-1680, the start and stop coordinates, on the contig 1610, for the first base and last base are determined. The mean and standard deviation of all of the start points for each sequence read 1650-1680 are determined, resulting in a mean start point and a start standard deviation. In a similar fashion, the mean and standard deviation of all of the stop points for each sequence read 1650-1680 are determined, resulting in a mean stop point and a stop standard deviation. The spread (1710, 1720) is then defined as the minimum, or lowest, standard deviation between the start standard deviation and the stop standard deviation. The threshold for the spread test may be from, and including, 1-15 bases. In an embodiment, the threshold may be 8 bases. If the spread (1710, 1720) is less than 8, then the fusion fails the spread test and it is discarded. In an embodiment, the threshold may be 7 bases. In an embodiment, the threshold may be 6 bases.

Returning to FIG. 4, determining fusion events at step 440 may comprise applying one or more criteria to the one or more candidate fusion events and determining, based on application of the one or more criteria, one or more fusion events. Any candidate fusion events remaining after application of the one or more criteria may be identified as fusion events.

The one or more criteria may comprise, for example, closeness of the candidate fusion event to a probe. At least one candidate fusion event (e.g., breakpoint) must be within a distance of a probe used in an enrichment step of the sample or else the candidate fusion event is discarded. By way of example, the distance may be anywhere from, and including, 250 to 500 bases. In an embodiment, the distance may be 300 bases. In an embodiment, the distance may be 350 bases. In an embodiment, the distance may be 400 bases. In an embodiment, the distance may be 450 bases.

The one or more criteria may comprise, for example, application of a whitelist. A whitelist of genes may be determined. If a candidate fusion event (e.g., breakpoint) is not associated with one of the genes in the whitelist, the candidate fusion event is discarded.

The one or more criteria may comprise, for example, application of a blacklist. A blacklist of genes may be determined. If a candidate fusion event (e.g., breakpoint) is associated with one of the genes in the blacklist, the candidate fusion event is discarded.

The one or more criteria may comprise, for example, filtering certain indels. If a candidate fusion event (e.g., breakpoint) is an indel that is completely embedded in an intronic region, the candidate fusion event is discarded. If a candidate fusion event (e.g., breakpoint) is a deletion and is shorter than a threshold number of bases, the candidate fusion event is discarded. The threshold number of bases may be anywhere from, and including, 10 to 100 bases. In an embodiment, the threshold number of bases may be 50 bases. If a candidate fusion event (e.g., breakpoint) is a deletion and is within a threshold distance of another deletion, the candidate fusion event is discarded. The threshold distance may be anywhere from, and including, 10 to 100 bases. In an embodiment, the threshold distance may be 49 bases. In an embodiment, the threshold distance may be 48 bases. In an embodiment, the threshold distance may be 47 bases. In an embodiment, the threshold distance may be 46 bases. In an embodiment, the threshold distance may be 45 bases.

The one or more criteria may comprise, for example, determining if a ratio of molecules to reads exceeds a threshold and there are no double stranded supporting molecules (a double stranded supporting molecule being defined as a molecule with 2 or more reads on each strand). The threshold may be anywhere from, and including, .5 to .9. In an embodiment, the threshold may be .8. In an embodiment, the threshold may be .7. In an embodiment, the threshold may be .6. In an embodiment, the threshold may be .5. If the ratio associated with a candidate fusion event is greater than and/or equal to the threshold, the candidate fusion event is discarded.

The one or more criteria may comprise, for example, determining that the candidate fusion event is a stitching artifact. A stitching artifact may be a long molecule that has been stitched across a short repeat (introducing an artificial deletion event). The stitching process may fuse long molecules at a perfect repeat, resulting in a stitching artifact that may be classified as a candidate fusion event. As shown in FIG. 3, neighboring perfect repeats on two sequence reads may cause long molecules to be stitched incorrectly. To address this issue, a number of bases of the reference sequence abutting the breakpoints may be aligned against each other, and the candidate fusion event may be discarded if the alignment score is greater than or equal to a threshold score. The number of bases may be anywhere from, and including, 80 to 160. In an embodiment, the number of bases may be 120. The threshold score may be anywhere from, and including, 60 to 80. In an embodiment, the threshold score may be 70.

The one or more criteria may comprise, for example, determining that the candidate fusion event is an template switching artifact. A template switch is an artifact that occurs in during sequence library preparation because of sequence similarity. This issue is similar to stitching artifacts. To address this issue a number of bases of the reference centered around the two breakpoints may be aligned against each other, and the candidate fusion event may be discarded if the alignment score is greater than or equal to a threshold score. The threshold score may be anywhere from, and including, 10 to 30. In an embodiment, the threshold score may be 20.

Determining an alignment score is well known in the art. Sequence alignment can use an algorithm to establish similarity between two sequences. For example, a positive number can be assigned for each match of the sequences and a negative number can be assigned for each mismatch of the sequences. The sum of these numbers can then be used as the alignment score. Programs such as Basic Local Alignment Search Tool (BLAST), MUSCLE, Mauve, MAFFT, Clustal Omega, Jotun Hein, Wilbur-Lipman, Martinez Needleman-Wunsch, Lipman-Pearson, Kalign, MView, and EMBOSS Cons can be used to determine an alignment score.

The one or more criteria may comprise, for example, determining that the candidate fusion event contains a suitable number of non-singleton supporting molecules. A singleton supporting molecule is a sequence molecule with family size of one, and the suitability test may check for the existence of one or more non-singleton molecules, or for the existence of two or more non-singleton molecules, or for the existence of a predefined number or more of non-singleton molecules.

The aforementioned methods and systems for determining fusion events differ from typical techniques that rely solely on alignment of input reads against a reference genome to identify discordant alignments that may be the result of fusion events. When relying on alignment alone, once a fusion supporting read is misaligned , it can no longer be recovered downstream, thereby leading to false positive fusion calls. Moreover, the present methods and systems can quickly and accurately identify a fusion event, and reduce time and complexity as compared to previous systems.

Fusion detection is an important aspect of an oncology pipeline. Tumors are known to rearrange portions of genomes to either enhance the function of genes it needs, or to suppress the functionality of tumor suppressor genes. Some drugs are specifically designed to address certain tumors driven by certain fusions. The identification of these fusions has a significant impact on treatment identification and treatment selection for a given patient.

The methods and systems described generate clinically relevant gene fusion data containing low false-positive gene fusion detections based on a subject's DNA sequence information (DNA-SEQ) and/or RNA sequence information (RNA-SEQ) data sets. The resultant annotated gene fusion data contains clinically relevant information and high specificity gene fusion identification (e.g., low false-positives) that can be used in clinical and/or R&D settings.

Disclosed are methods of using the information (e.g. identification of fusion events) determined in the disclosed methods. For example, a subject may be treated by administering a cancer therapeutic to the subject, wherein the subject has been determined to have a fusion event using one or more of the disclosed methods. In some aspects, the subject has been determined to have cancer based on the identification of a fusion event using one or more of the disclosed methods. In some aspects, the cancer can be any cancer associated with a fusion event. Cancers associated with a fusion event can be any cancer caused by a fusion event. For example, cancers associated with fusion events can be, but are not limited to, advanced urothelial cancer, prostate cancer, breast cancer, lung cancer, colon cancer, glioblastoma, liver cancer, or ovarian cancer. In some aspects, the cancer therapeutic can be a known cancer therapeutic used for treating a specific cancer. For example, if the subject is determined to have an FGFR2/3 fusion event then the FDA-approved drug, erdafitinib, can be administered to the subject. Thus, in some aspects, the cancer therapeutic is specific to the fusion event. A cancer therapeutic specific to a fusion event can be a cancer therapeutic previously determined to effectively treat a cancer associated with the specific fusion event.

In some aspects, a subject can be previously diagnosed with cancer (prior to knowledge of a fusion event) and then upon identification of a fusion event using the disclosed methods, a specific cancer therapeutic can be administered to the subject. Thus, identification of a fusion event using the disclosed methods can allow for personalized medicine.

Performance evaluation of the disclosed methods and systems was performed relying on proxies. The proxies include AV samples and samples from healthy donors. An existing production pipeline software package, having a fusion caller function, has been thoroughly tested on a selected set of fusion events (not as a *de novo* caller). Abfusion's sensitivity is comparable to the sensitivity of the fusion caller function, which is however run only on a very limited set of fusion cases.

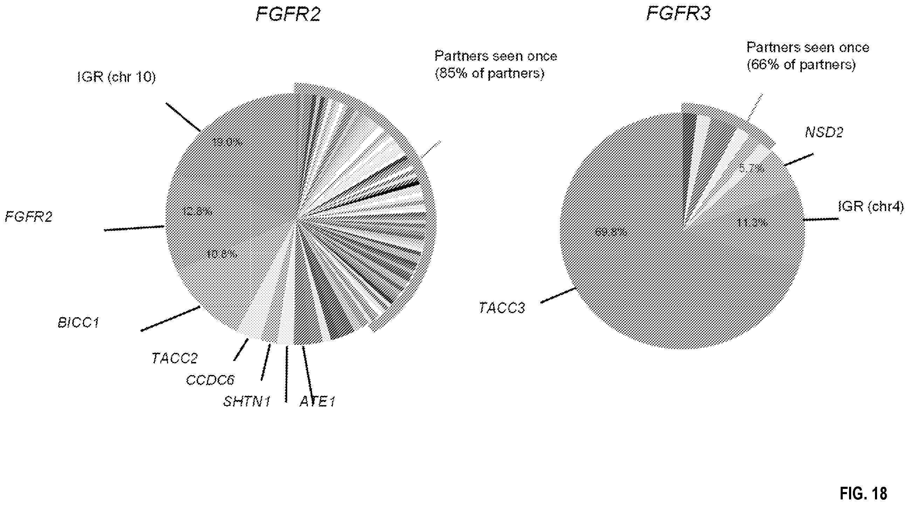

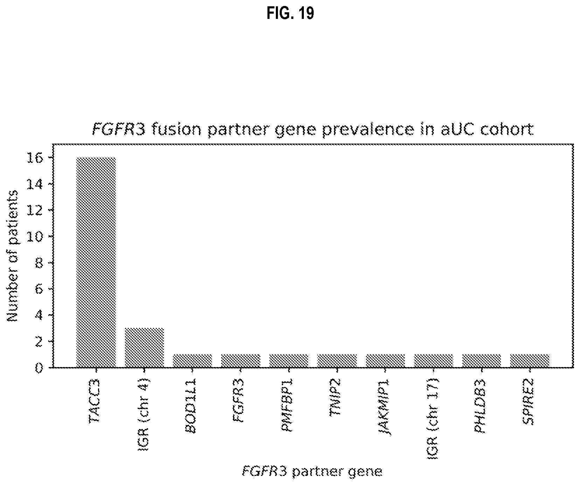

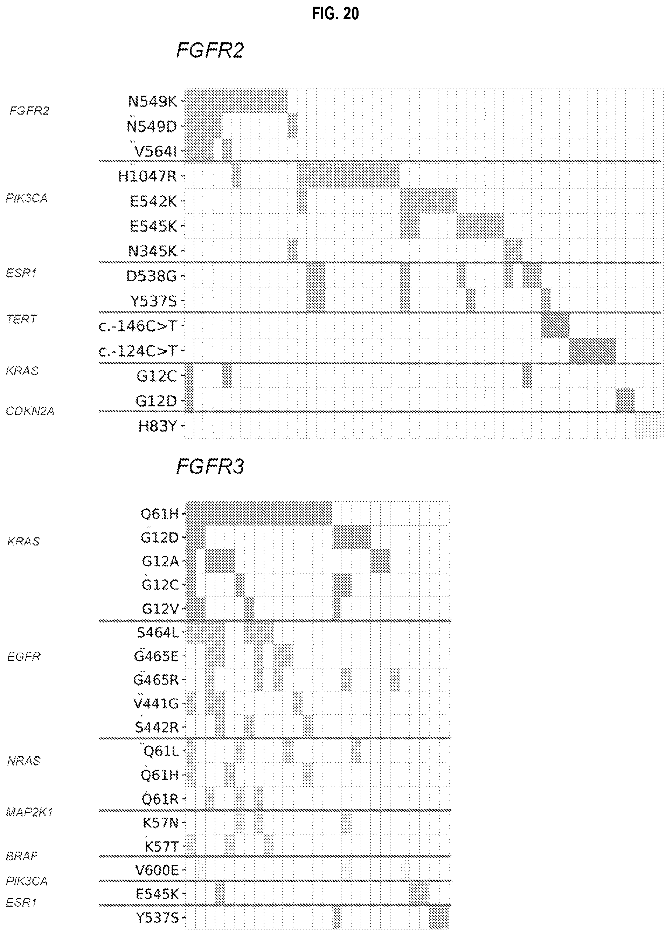

In one example, the *de novo* fusion caller was used to identify *FGFR2*/*3* fusions from clinical cfDNA. *FGFR2*/*3* rearrangements are therapeutic targets, especially in advanced urothelial cancer (aUC) with FDA-approved erdafitinib. Liquid biopsy is an attractive non-invasive method to identify these fusions, but detection in cfDNA is technically challenging due to low tumor shedding levels, short molecules, and wide variation in gene partners. To address this, the *de novo* fusion caller was used. A cohort of 17,718 patients with mixed cancer types (including 795 aUC patients, as well as breast, cholangiocarcinoma, colorectal, and gastric), plus 276 healthy control samples, that were previously tested on cfDNA NGS-based assay, were reanalyzed using the de novo fusion caller. The median unique molecule coverage was approximately 3,000 molecules sequenced to 15,000x read depth. Samples were reanalyzed in silico using the novel algorithm: in brief, reads aligned to candidate fusion breakpoints were assembled into de Bruijn graphs. Resulting contigs were aligned to the reference and filters were applied to remove technical artifacts. The majority of *FGFR2* (85%) and *FGFR3* fusion partners (66%) in the mixed cancer cohort were observed only once (FIG. 18), consistent with previous reports. *FGFR3-TACC3* was the most common fusion, occurring in 59% of *FGFR3* fusion-positive patients. In 36% of FGFR2 fusion positive patients, the *de novo* caller detected partners were not previously described. In the aUC cohort, *FGFR3* fusions were detected in 3.1% of patients, with 8/10 (80%) partner genes/intergenic regions occurring only once, which is in line with previous reports (FIG. 19). No fusions were identified in 276 healthy control samples. In the mixed cancer cohort, common mutations co-occurring with *FGFR2* fusions that were enriched in patients with these fusions were *FGFR2* N549K (7.1%), *FGFR2* N549D (3.2%), and *FGFR2* V564I (2.6%); common mutations co-occurring with *FGFR3* fusions that were enriched in patients with these fusions included *KRAS* Q61H, observed in 30.6% of patients with *FGFR3* fusions FIG. 20. Thus, the *FGFR3* fusion prevalence observed in cfDNA from aUC patients that is comparable to previous reports for tissue testing, demonstrate the ability to capture targetable genomic rearrangements with plasma-based NGS. FGFR2/3 fusion partners detected by a highly specific assembly-based de novo fusion caller were heterogeneous and individually rare, highlighting the importance of a de novo approach.

FIG. 21 is a block diagram depicting an environment 2100 comprising non-limiting examples of a computing device 2101 and servers 2102 connected through a network 2103. In an aspect, some or all steps of any described method may be performed on a computing device as described herein. The computing device 2101 can comprise one or multiple computers configured to store one or more of a fusion caller module 2104, sequence data 2105 (e.g., sequence reads, contigs, reference sequences, criteria, container data structures, graph data structures, etc.), and the like. The servers 2102 can comprise one or multiple computers configured to store a fusion caller module 2104, sequence data 2105 (e.g., sequence reads, contigs, reference sequences, criteria, etc...), and the like for remote access. Multiple servers 2102 can communicate with the computing device 2101 via the through the network 2103.

The computing device 2101 and the server 2102 can be a digital computer that, in terms of hardware architecture, generally includes a processor 2106, memory system 2107, input/output (I/O) interfaces 2108, and network interfaces 2109. These components (2106, 2107, 2108, and 2109) are communicatively coupled via a local interface 2110. The local interface 2110 can be, for example, but not limited to, one or more buses or other wired or wireless connections, as is known in the art. The local interface 2110 can have additional elements, which are omitted for simplicity, such as controllers, buffers (caches), drivers, repeaters, and receivers, to enable communications. Further, the local interface may include address, control, and/or data connections to enable appropriate communications among the aforementioned components.

The processor 2106 can be a hardware device for executing software, particularly that stored in memory system 2107. The processor 2106 can be any custom made or commercially available processor, a central processing unit (CPU), an auxiliary processor among several processors associated with the computing device 2101 and the server 2102, a semiconductor-based microprocessor (in the form of a microchip or chip set), or generally any device for executing software instructions. When the computing device 2101 and/or the server 2102 is in operation, the processor 2106 can be configured to execute software stored within the memory system 2107, to communicate data to and from the memory system 2107, and to generally control operations of the computing device 2101 and the server 2102 pursuant to the software.

The I/O interfaces 2108 can be used to receive user input from, and/or for providing system output to, one or more devices or components. User input can be provided via, for example, a keyboard and/or a mouse. System output can be provided via a display device and a printer (not shown). I/O interfaces 2108 can include, for example, a serial port, a parallel port, a Small Computer System Interface (SCSI), an infrared (IR) interface, a radio frequency (RF) interface, and/or a universal serial bus (USB) interface.

The network interface 2109 can be used to transmit and receive from the computing device 2101 and/or the server 2102 on the network 2103. The network interface 2109 may include, for example, a 10BaseT Ethernet Adaptor, a 100BaseT Ethernet Adaptor, a LAN PHY Ethernet Adaptor, a Token Ring Adaptor, a wireless network adapter (e.g., WiFi, cellular, satellite), or any other suitable network interface device. The network interface 2109 may include address, control, and/or data connections to enable appropriate communications on the network 2103.

The memory system 2107 can include any one or combination of volatile memory elements (e.g., random access memory (RAM, such as DRAM, SRAM, SDRAM, etc.)) and nonvolatile memory elements (e.g., ROM, hard drive, tape, CDROM, DVDROM, etc.). Moreover, the memory system 2107 may incorporate electronic, magnetic, optical, and/or other types of storage media. Note that the memory system 2107 can have a distributed architecture, where various components are situated remote from one another, but can be accessed by the processor 2106.

The software in memory system 2107 may include one or more software programs, each of which comprises an ordered listing of executable instructions for implementing logical functions. In the example of FIG. 21, the software in the memory system 2107 of the computing device 2101 can comprise the fusion caller module 2104 (or subcomponents thereof), the sequence data 2105, and a suitable operating system (O/S) 2111. The operating system 2111 essentially controls the execution of other computer programs and provides scheduling, input-output control, file and data management, memory management, and communication control and related services.

For purposes of illustration, application programs and other executable program components such as the operating system 2111 are illustrated herein as discrete blocks, although it is recognized that such programs and components can reside at various times in different storage components of the computing device 2101 and/or the servers 2102. An implementation of the fusion caller module 2104 can be stored on or transmitted across some form of computer readable media. Any of the disclosed methods can be performed by computer readable instructions embodied on computer readable media. Computer readable media can be any available media that can be accessed by a computer. By way of example and not meant to be limiting, computer readable media can comprise "computer storage media" and "communications media." "Computer storage media" can comprise volatile and non-volatile, removable and non-removable media implemented in any methods or technology for storage of information such as computer readable instructions, data structures, program modules, or other data. Exemplary computer storage media can comprise RAM, ROM, EEPROM, flash memory or other memory technology, CD-ROM, digital versatile disks (DVD) or other optical storage, magnetic cassettes, magnetic tape, magnetic disk storage or other magnetic storage devices, or any other medium which can be used to store the desired information and which can be accessed by a computer.

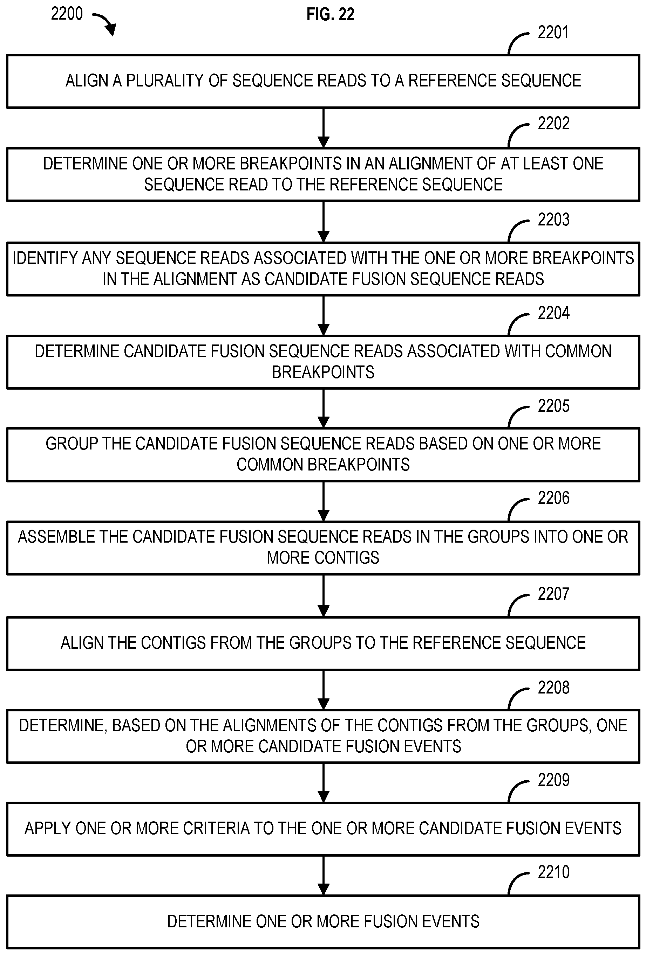

In an embodiment, the fusion caller module 2104 may be configured to access the sequence data 2105 and perform a method 2200, shown in FIG. 22. The method 2200 may be performed in whole or in part by a single computing device, a plurality of electronic devices, and the like. The method 2200 may comprise aligning a plurality of sequence reads to a reference sequence at step 2201.

The method 2200 may comprise determining one or more breakpoints in an alignment of at least one sequence read of the plurality of sequence reads to the reference sequence at step 2202.

The method 2200 may comprise identifying any sequence reads associated with the one or more breakpoints in the alignment as candidate fusion sequence reads at step 2203. Identifying any sequence reads associated with the one or more breakpoints in the alignment as candidate fusion sequence reads can comprise discarding alignments have a mappability score below a threshold. Identifying any sequence reads associated with the one or more breakpoints in the alignment as candidate fusion sequence reads can comprise discarding alignments that are logical.

The method 2200 may comprise determining candidate fusion sequence reads associated with common breakpoints of one or more breakpoints at step 2204. Determining candidate fusion sequence reads associated with common breakpoints of one or more breakpoints can comprise determining that two candidate fusion sequence reads comprise a breakpoint in a same chromosome and at a same orientation. Determining candidate fusion sequence reads associated with common breakpoints of one or more breakpoints can comprise determining that two candidate fusion sequence reads comprise a breakpoint at a same position. Determining candidate fusion sequence reads associated with common breakpoints of one or more breakpoints can comprise determining that two candidate fusion sequence reads comprise a breakpoint within a threshold number of bases from a position. The threshold number of bases from the position may be, for example, 1-40 bases. In an embodiment, the threshold number of bases from the position may be 10 bases. In an embodiment, the threshold number of bases from the position may be 11 bases. In an embodiment, the threshold number of bases from the position may be 12 bases. Determining candidate fusion sequence reads associated with common breakpoints of one or more breakpoints can comprise determining that two candidate fusion sequence reads comprise a plurality of breakpoints in a same chromosome and at a same orientation. Determining candidate fusion sequence reads associated with common breakpoints of one or more breakpoints can comprise determining that two candidate fusion sequence reads comprise a plurality of breakpoints at same positions. Determining candidate fusion sequence reads associated with common breakpoints of one or more breakpoints can comprise determining that two candidate fusion sequence reads comprise a plurality of breakpoints within a threshold number of bases from a plurality of positions. The threshold number of bases from the plurality of positions may be, for example, 1-40 bases. In an embodiment, the threshold number of bases from the plurality of positions may be 10 bases. In an embodiment, the threshold number of bases from the plurality of positions may be 11 bases. In an embodiment, the threshold number of bases from the plurality of positions may be 12 bases. In an embodiment, the threshold number of bases from the plurality of positions may be 13 bases. In an embodiment, the threshold number of bases from the plurality of positions may be 14 bases. In an embodiment, the threshold number of bases from the plurality of positions may be 15 bases.

The method 2200 may comprise grouping the candidate fusion sequence reads based on one or more common breakpoints at step 2205. Grouping the candidate fusion sequence reads based on one or more common breakpoints can comprise generating a de Bruijn graph for the groups (e.g., for each group).

The method 2200 may comprise assembling the candidate fusion sequence reads in the groups (e.g., for each group) into one or more contigs at step 2206. Assembling the candidate fusion sequence reads in the groups into one or more contigs can comprise linearizing each de Bruijn graph to generate a contig for the groups. Assembling the candidate fusion sequence reads in the groups into one or more contigs can comprise performing one or more error correction procedures. The one or more error correction procedures can comprise resolving mismatches between candidate fusion sequence reads and the reference sequence. The one or more error correction procedures can comprise inserting padding between at least two candidate fusion sequence reads. The one or more error correction procedures can comprise discarding one or more candidate fusion sequence reads having an unaligned portion that exceeds a threshold.

The method 2200 may comprise aligning the contigs from the groups (e.g., for each group) to the reference sequence at step 2207.

The method 2200 may comprise determining, based on the alignments of the contigs from the groups (e.g., for each group), one or more candidate fusion events at step 2208. Determining, based on the alignments of the contigs from the groups, one or more candidate fusion events can comprise applying one or more of a footprint test or a spread test. Applying the footprint test can comprise determining that a threshold number of families of candidate fusion sequence reads that support the contig span the breakpoint(s). Applying the spread test comprises determining that a threshold amount of spread exists between at least two families of candidate fusion sequence reads that support the contig and span the breakpoint(s).

The method 2200 may comprise applying one or more criteria to the one or more candidate fusion events at step 2209.

Applying one or more criteria to the one or more candidate fusion events can comprise determining, for the candidate fusion events (e.g., for each candidate fusion event), a distance between a breakpoint of the one or more aligned contigs and a location of at least one probe of a panel and discarding any candidate fusion event associated with an aligned contig of the one or more contigs containing no breakpoint with a distance from the location of at least one probe of a panel less than a threshold. By way of example, the distance may be, from 1-1,000 bases. In an embodiment, the distance may be 350 bases. The sequence reads (step 2201), from which the candidate fusion events are determined, may be derived from DNA that has been enriched for the panel.

Applying one or more criteria to the one or more candidate fusion events can comprise determining one or more genes of interest and discarding any candidate fusion event associated with an aligned contig of the one or more contigs containing no breakpoint that is associated with the one or more genes of interest.

Applying one or more criteria to the one or more candidate fusion events can comprise determining, for the candidate fusion events, that a breakpoint of the one or more aligned contigs is a deletion and discarding any candidate fusion event associated with an aligned contig of the one or more contigs comprising a deletion located within a number of bases away from another deletion.

Applying one or more criteria to the one or more candidate fusion events can comprise determining, for the candidate fusion events, that a breakpoint of the one or more aligned contigs is a deletion and discarding any candidate fusion event associated with an aligned contig of the one or more contigs comprising a deletion comprising a number of bases less than a threshold.

Applying one or more criteria to the one or more candidate fusion events can comprise discarding any candidate fusion event associated with an aligned contig of the one or more contigs comprising an insertion or a deletion that is completely embedded in an intronic region.

Applying one or more criteria to the one or more candidate fusion events can comprise determining, for the candidate fusion events, for the one or more aligned contigs, a ratio of molecules to reads and discarding any candidate fusion event associated with an aligned contig of the one or more contig that is associated with a ratio of molecules to reads greater than a threshold and that is not associated with a double stranded supporting molecule.

Applying one or more criteria to the one or more candidate fusion events can comprise determining, for the candidate fusion events, for the pairs of breakpoints of the one or more aligned contigs, a sequence abutting the breakpoint of the pair of breakpoints, aligning the sequences abutting the breakpoint of the pair of breakpoints, determining an alignment score for the alignment of the sequences abutting the breakpoint of the pair of breakpoints, and discarding any candidate fusion event associated with an aligned contig of the one or more contigs based on the alignment score exceeding a threshold.

Applying one or more criteria to the one or more candidate fusion events can comprise determining, for the candidate fusion events, for the pairs of breakpoints of the one or more aligned contigs, a sequence centered on the breakpoints of the pair of breakpoints, aligning the sequences centered around the breakpoint against each other, determining an alignment score for the alignment of the sequences centered around the breakpoints, and discarding any candidate fusion event associated with an aligned contig of the one or more contigs based on the alignment score exceeding a threshold.

The method 2200 may comprise determining, based on applying the one or more criteria to the one or more candidate fusion events, one or more fusion events at step 2210. Any remaining candidate fusion events may be determined as the one or more fusion events.

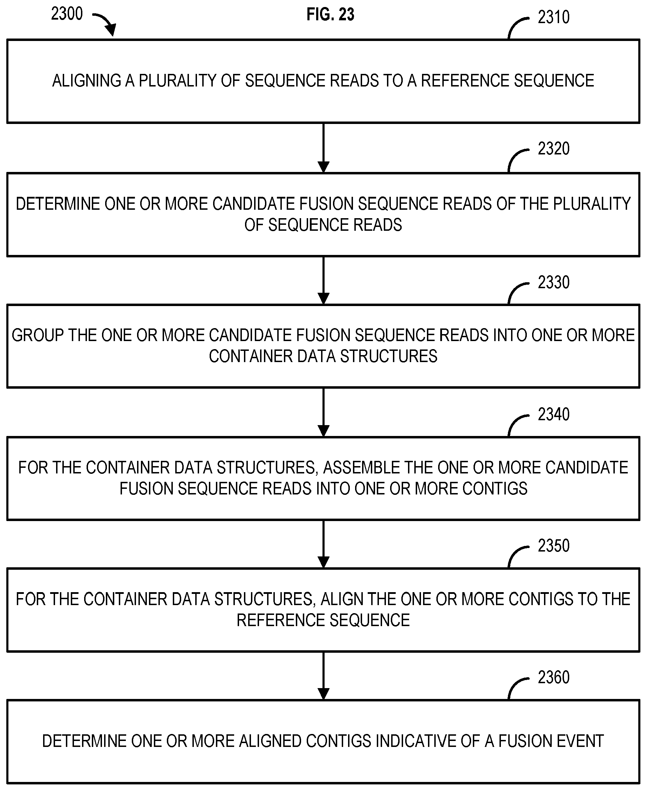

In an embodiment, the fusion caller module 2104 may be configured to access the sequence data 2105 and perform a method 2300, shown in FIG. 23. The method 2300 may be performed in whole or in part by a single computing device, a plurality of electronic devices, and the like. The method 2300 may comprise aligning a plurality of sequence reads to a reference sequence at step 2310.

The method 2300 may comprise determining, based on one or more breakpoints in the alignments of a sequence read to the reference sequence, one or more candidate fusion sequence reads of the plurality of sequence reads at step 2320. Determining, based on one or more breakpoints in the alignments of a sequence read to the reference sequence, one or more candidate fusion sequence reads of the plurality of sequence reads can comprise determining that two candidate fusion sequence reads comprise a breakpoint in a same chromosome and at a same orientation. Determining, based on one or more breakpoints in the alignments of a sequence read to the reference sequence, one or more candidate fusion sequence reads of the plurality of sequence reads can comprise determining that two candidate fusion sequence reads comprise a breakpoint at a same position. Determining, based on one or more breakpoints in the alignments of a sequence read to the reference sequence, one or more candidate fusion sequence reads of the plurality of sequence reads can comprise determining that two candidate fusion sequence reads comprise a breakpoint within a threshold number of bases from a position. The threshold number of bases from the position may be, for example, 1-40 bases. In an embodiment, the threshold number of bases from the position may be 10 bases. In an embodiment, the threshold number of bases from the position may be 11 bases. In an embodiment, the threshold number of bases from the position may be 12 bases. Determining, based on one or more breakpoints in the alignments of a sequence read to the reference sequence, one or more candidate fusion sequence reads of the plurality of sequence reads can comprise determining that two candidate fusion sequence reads comprise a plurality of breakpoints in a same chromosome and at a same orientation. Determining, based on one or more breakpoints in the alignments of a sequence read to the reference sequence, one or more candidate fusion sequence reads of the plurality of sequence reads can comprise determining that two candidate fusion sequence reads comprise a plurality of breakpoints at same positions. Determining, based on one or more breakpoints in the alignments of a sequence read to the reference sequence, one or more candidate fusion sequence reads of the plurality of sequence reads can comprise determining that two candidate fusion sequence reads comprise a plurality of breakpoints within a threshold number of bases from a plurality of positions. The threshold number of bases from the plurality of positions may be, for example, 1-40 bases. In an embodiment, the threshold number of bases from the position may be 10 bases. In an embodiment, the threshold number of bases from the position may be 11 bases. In an embodiment, the threshold number of bases from the plurality of positions may be 12 bases.

The method 2300 may comprise grouping, based on one or more common breakpoints, the one or more candidate fusion sequence reads into one or more container data structures at step 2330. Breakpoints from different alignments may be assigned to a common container data structure. The one or more candidate fusion sequence reads into one or more container data structures according to a de Bruijn graph technique.

The method 2300 may comprise for the container data structures (e.g., for each container data structure), assembling the one or more candidate fusion sequence reads into one or more contigs at step 2340. Assembling the one or more candidate fusion reads into one or more contigs can comprise for the container data structures (e.g., for each container data structure), assembling the one or more candidate fusion sequence reads into a graph data structure and linearizing the graph data structure to generate one or more contigs. Assembling the one or more candidate fusion sequence reads into one or more contigs can comprise performing one or more error correction procedures. The one or more error correction procedures can comprise resolving mismatches between candidate fusion sequence reads and the reference sequence. The one or more error correction procedures can comprise inserting padding between two or more candidate fusion sequence reads. The one or more error correction procedures can comprise discarding one or more candidate fusion sequence reads having an unaligned portion that exceeds a threshold.

The method 2300 may comprise for the container data structures (e.g., for each container data structure), aligning the one or more contigs to the reference sequence at step 2350. The method 2300 may further comprise determining, based on the alignments of the contigs from the container data structures, one or more candidate fusion events can comprise applying one or more of a footprint test or a spread test. Applying the footprint test can comprise determining that a threshold number of families of candidate fusion sequence reads that support the contig span the breakpoint(s). Applying the spread test comprises determining that a threshold amount of spread exists between at least two families of candidate fusion sequence reads that support the contig and span the breakpoint(s).

The method 2300 may comprise determining, based on one or more criteria, one or more aligned contigs indicative of a fusion event at step 2360. Any remaining candidate fusion events may be determined as the one or more fusion events. Determining, based on the one or more criteria, the one or more aligned contigs indicative of one or more fusion events can comprise determining a distance between a breakpoint of the one or more aligned contigs and a location of at least one probe of a panel and discarding any aligned contig of the one or more contigs containing no breakpoint with a distance from the location of at least one probe of a panel less than a threshold. By way of example, the distance may be, from 1-1,000 bases. In an embodiment, the distance may be 350 bases. The sequence reads (step 2310), from which the candidate fusion events are determined, may be derived from DNA that has been enriched for the panel. Determining, based on the one or more criteria, the one or more aligned contigs indicative of the fusion event can comprise determining one or more genes of interest and discarding any aligned contig of the one or more contigs containing no breakpoint that is associated with the one or more genes of interest. Determining, based on the one or more criteria, the one or more aligned contigs indicative of the fusion event can comprise determining that a breakpoint of the one or more aligned contigs is a deletion and discarding any aligned contig of the one or more contigs comprising a deletion located within a number of bases away from another deletion. Determining, based on the one or more criteria, the one or more aligned contigs indicative of the fusion event can comprise determining that a breakpoint of the one or more aligned contigs is a deletion and discarding any aligned contig of the one or more contigs comprising a deletion comprising a number of bases less than a threshold. Determining, based on the one or more criteria, the one or more aligned contigs indicative of the fusion event can comprise discarding any aligned contig of the one or more contigs comprising an insertion or a deletion that is completely embedded in an intronic region. Determining, based on the one or more criteria, the one or more aligned contigs indicative of the fusion event can comprise determining, for the one or more aligned contigs, a ratio of molecules to reads and discarding any aligned contig of the one or more contig that is associated with a ratio of molecules to reads greater than a threshold and that is not associated with a double stranded supporting molecule. Determining, based on the one or more criteria, the one or more aligned contigs indicative of the fusion event can comprise determining, for the pairs of breakpoints of the one or more aligned contigs, a sequence abutting the breakpoints of the pair of breakpoints, aligning the sequences abutting the breakpoints of the pair of breakpoints, determining an alignment score for the alignment of the sequences abutting the breakpoints of the pair of breakpoints, and discarding any aligned contig of the one or more contigs based on the alignment score exceeding a threshold. Determining, based on the one or more criteria, the one or more aligned contigs indicative of the fusion event can comprise determining, for the pair of breakpoints of the one or more aligned contigs, a sequence centered on the breakpoints of the pair of breakpoints, aligning the sequences centered around the breakpoints against each other, determining an alignment score for the alignment of the sequences centered around the breakpoints, and discarding any aligned contig of the one or more contigs based on the alignment score exceeding a threshold.

The method 2300 may further comprise generating, based on discarding any aligned contig of the one or more contigs, a notification indicative of an issue associated with library preparation.

While specific configurations have been described, it is not intended that the scope be limited to the particular configurations set forth, as the configurations herein are intended in all respects to be possible configurations rather than restrictive. Unless otherwise expressly stated, it is in no way intended that any method set forth herein be construed as requiring that its steps be performed in a specific order. Accordingly, where a method claim does not actually recite an order to be followed by its steps or it is not otherwise specifically stated in the claims or descriptions that the steps are to be limited to a specific order, it is in no way intended that an order be inferred, in any respect. This holds for any possible non-express basis for interpretation, including: matters of logic with respect to arrangement of steps or operational flow; plain meaning derived from grammatical organization or punctuation; the number or type of configurations described in the specification.

It will be apparent to those skilled in the art that various modifications and variations may be made. Other configurations will be apparent to those skilled in the art from consideration of the specification and practice described herein. It is intended that the specification and described configurations be considered as exemplary only.

## Claims

1. A computer-implemented method comprising:
aligning a plurality of sequence reads to a reference sequence;
determining one or more breakpoints in an alignment of a plurality of sequence reads of the plurality of sequence reads to the reference sequence;
identifying any sequence reads associated with the one or more breakpoints in the alignment as candidate fusion sequence reads;
determining candidate fusion sequence reads associated with common breakpoints of one or more breakpoints;
grouping the candidate fusion sequence reads based on one or more common breakpoints;
assembling the candidate fusion sequence reads in the groups into one or more contigs;
aligning the contigs from the groups of the plurality of groups to the reference sequence;
determining, based on the alignments of the contigs from the groups, one or more candidate fusion events, wherein the determining comprises:
aligning candidate fusion sequence reads for the group to the contig,
clustering the candidate fusion sequence reads into families of reads associated with the same molecule, and applying a spread test to the alignments; wherein applying the spread test comprises determining that a threshold amount of spread exists between sequence reads of at least two families of candidate fusion sequence reads that support the contig and span the breakpoint(s); wherein the spread is defined as the minimum, or lowest, standard deviation between a standard deviation of read start points on the contig and a standard deviation of read stop points on the contig;
applying one or more criteria to the one or more candidate fusion events; and
determining, based on applying the one or more criteria to the one or more candidate fusion events, one or more fusion events.

2. The method of claim 1, wherein identifying any sequence reads associated with the one or more breakpoints in the alignment as candidate fusion sequence reads comprises
(i) discarding alignments that have a mappability score below a threshold;
and/or
(ii) discarding alignments that are logical.

3. The method of claim 1 or claim 2, wherein determining candidate fusion sequence reads associated with common breakpoints of one or more breakpoints comprises
(i) determining that at least two candidate fusion sequence reads comprise a breakpoint in a same chromosome and at a same orientation;
(ii) determining that at least two candidate fusion sequence reads comprise a breakpoint at a same position;
(iii) determining that at least two candidate fusion sequence reads comprise a breakpoint within a threshold number of bases from a position;
(iv) determining that at least two candidate fusion sequence reads comprise a plurality of breakpoints in a same chromosome and at a same orientation;
(v) determining that at least two candidate fusion sequence reads comprise a plurality of breakpoints at same positions; and/or
(vi) determining that at least two candidate fusion sequence reads each comprise a plurality of breakpoints within a threshold number of bases from a plurality of positions.

4. The method of any one of the preceding claims, wherein grouping the candidate fusion sequence reads based on one or more common breakpoints comprises generating a de Bruijn graph for the groups, for example for each group; optionally wherein assembling the candidate fusion sequence reads in the groups into one or more contigs comprises linearizing the de Bruijn graphs to generate a contig for the groups.

5. The method of any one of the preceding claims, wherein assembling the candidate fusion sequence reads in the groups into one or more contigs comprises performing one or more error correction procedures; for example wherein the one or more error correction procedures comprises
(i) resolving mismatches between candidate fusion sequence reads and the reference sequence;
(ii) inserting padding between at least two candidate fusion sequence reads;
and/or
(iii) discarding one or more candidate fusion sequence reads having an unaligned portion that exceeds a threshold.

6. The method of any one of the preceding claims, wherein aligning the contigs from the groups of the plurality of groups to the reference sequence comprises aligning to a reference sequence with decoys.

7. The method of any one of the preceding claims, wherein a family is determined based on molecular barcoding, for example wherein
(i) candidate fusion sequence reads containing the same molecular barcode are grouped into the same family; and/or
(ii) sequence reads containing the same molecular barcode and whose alignments begin within a number of bases, for example 30-50 bases, of each other are grouped into the same family.

8. The method of any one of the preceding claims, wherein determining, based on the alignments of the contigs from the groups, one or more candidate fusion events further comprises applying a footprint test, wherein applying the footprint test comprises determining that a threshold number of families of candidate fusion sequence reads that support the contig span the breakpoint(s); for example wherein the threshold is anywhere from, and including, 2 to 5 families.

9. The method of any one of the preceding claims, wherein the threshold for the spread test is 8 bases, 7 bases, 6 bases, or 5 bases.

10. The method of any one of the preceding claims, wherein applying one or more criteria to the one or more candidate fusion events comprises:
(i) determining, for the candidate fusion events, a distance between a breakpoint of the one or more aligned contigs and a location of at least one probe of a panel; and
discarding any candidate fusion event associated with an aligned contig of the one or more contigs containing no breakpoint with a distance from the location of at least one probe of a panel less than a threshold;
(ii) determining one or more genes of interest; and
discarding any candidate fusion event associated with an aligned contig of the one or more contigs containing no breakpoint that is associated with the one or more genes of interest;
(iii) determining, for the candidate fusion events, that a breakpoint of the one or more aligned contigs is a deletion; and
discarding any candidate fusion event associated with an aligned contig of the one or more contigs comprising a deletion located within a number of bases away from another deletion;
(iv) determining, for the candidate fusion events, that a breakpoint of the one or more aligned contigs is a deletion; and
discarding any candidate fusion event associated with an aligned contig of the one or more contigs comprising a deletion comprising a number of bases less than a threshold;
(v) discarding any candidate fusion event associated with an aligned contig of the one or more contigs comprising an insertion or a deletion that is completely embedded in an intronic region;
(vi) determining, for the candidate fusion event, for the one or more aligned contigs, a ratio of molecules to reads; and
discarding any candidate fusion event associated with an aligned contig of the one or more contig that is associated with a ratio of molecules to reads greater than a threshold and that is not associated with a double stranded supporting molecule;
(vii) determining, for the candidate fusion event, for the pairs of breakpoints of the one or more aligned contigs, a sequence abutting the breakpoints of the pair of breakpoints;
aligning the sequences abutting the breakpoints of the pair of breakpoints;
determining an alignment score for the alignment of the sequences abutting the breakpoints of the pair of breakpoints; and
discarding any candidate fusion event associated with an aligned contig of the one or more contigs based on the alignment score exceeding a threshold; and/or
(viii) determining, for the candidate fusion events, for the pairs of breakpoints of the one or more aligned contigs, a sequence centered on the breakpoints of the pair of breakpoints;
aligning the sequences centered around the breakpoints against each other;
determining an alignment score for the alignment of the sequences centered around the breakpoints; and
discarding any candidate fusion event associated with an aligned contig of the one or more contigs based on the alignment score exceeding a threshold.

11. The method of any one of the preceding claims, further comprising generating a report, for example wherein the fusion events are displayed in the report and/or wherein the report comprises diagnostic information or therapeutic recommendations based on the determination of the fusion events.

12. An apparatus comprising:
one or more processors; and
memory storing processor executable instructions that, when executed by the one or more processors, cause the apparatus to perform the methods of any of claims 1-11.

13. A non-transitory computer-readable medium storing processor executable instructions that, when executed by at least one computing device, cause the at least one computing device to perform the methods of any of claims 1-11.

14. A system comprising at least one computing device configured to perform the methods of any of claims 1-11.

## Patentansprüche

1. Computerimplementiertes Verfahren, umfassend:
Alignieren einer Vielzahl von Sequenzlesungen an einer Referenzsequenz;
Bestimmen eines oder mehrerer Bruchpunkte in einem Alignment einer Vielzahl von Sequenzlesungen der Vielzahl von Sequenzlesungen an der Referenzsequenz;
Identifizieren jedweder Sequenzlesungen, die mit dem einen oder den mehreren Bruchpunkten in dem Alignment assoziiert sind, als Kandidatenfusionssequenzlesungen;
Bestimmen von Kandidatenfusionssequenzlesungen, die mit gemeinsamen Bruchpunkten von einem oder mehreren Bruchpunkten assoziiert sind;
Gruppieren der Kandidatenfusionssequenzlesungen basierend auf einem oder mehreren gemeinsamen Bruchpunkten;
Zusammensetzen der Kandidatenfusionssequenzlesungen in den Gruppen zu einem oder mehreren Contigs;
Alignieren der Contigs aus den Gruppen der Vielzahl von Gruppen an der Referenzsequenz;
Bestimmen, basierend auf den Alignments der Contigs aus den Gruppen, eines oder mehrerer Kandidatenfusionsereignisse, wobei das Bestimmen Folgendes umfasst:
Alignieren von Kandidatenfusionssequenzlesungen für die Gruppe an dem Contig, Clustern der Kandidatenfusionssequenzlesungen zu Familien von Lesungen, die mit demselben Molekül assoziiert sind, und Anwenden eines Spread-Tests auf die Alignments; wobei das Anwenden des Spread-Tests das Bestimmen umfasst, dass ein Spread-Schwellenwertbetrag zwischen Sequenzlesungen von mindestens zwei Familien von Kandidatenfusionssequenzlesungen existiert, die das Contig stützen und die Bruchpunkt(e) überspannen; wobei der Spread als die minimale oder niedrigste Standardabweichung zwischen einer Standardabweichung von Lesestartpunkten auf dem Contig und einer Standardabweichung von Lesestopppunkten auf dem Contig definiert ist;
Anwenden eines oder mehrerer Kriterien auf das eine oder die mehreren Kandidatenfusionsereignisse; und
Bestimmen, basierend auf dem Anwenden des einen oder der mehreren Kriterien auf das eine oder die mehreren Kandidatenfusionsereignisse, eines oder mehrerer Fusionsereignisse.

2. Verfahren nach Anspruch 1, wobei das Identifizieren jedweder Sequenzlesungen, die mit dem einen oder den mehreren Bruchpunkten in dem Alignment assoziiert sind, als Kandidatenfusionssequenzlesungen Folgendes umfasst
(i) Verwerfen von Alignments, die eine Kartierungsbewertung unter einem Schwellenwert aufweisen; und/oder
(ii) Verwerfen von Alignments, die logisch sind.

3. Verfahren nach Anspruch 1 oder Anspruch 2, wobei das Bestimmen von Kandidatenfusionssequenzlesungen, die mit gemeinsamen Bruchpunkten von einem oder mehreren Bruchpunkten assoziiert sind, Folgendes umfasst
(i) Bestimmen, dass mindestens zwei Kandidatenfusionssequenzlesungen einen Bruchpunkt in einem gleichen Chromosom und in einer gleichen Orientierung umfassen;
(ii) Bestimmen, dass mindestens zwei Kandidatenfusionssequenzlesungen einen Bruchpunkt an einer gleichen Position umfassen;
(iii) Bestimmen, dass mindestens zwei Kandidatenfusionssequenzlesungen einen Bruchpunkt innerhalb einer Schwellenanzahl von Basen von einer Position aus umfassen;
(iv) Bestimmen, dass mindestens zwei Kandidatenfusionssequenzlesungen eine Vielzahl von Bruchpunkten in einem gleichen Chromosom und in einer gleichen Orientierung umfassen;
(v) Bestimmen, dass mindestens zwei Kandidatenfusionssequenzlesungen eine Vielzahl von Bruchpunkten an gleichen Positionen umfassen; und/oder
(vi) Bestimmen, dass mindestens zwei Kandidatenfusionssequenzlesungen jeweils eine Vielzahl von Bruchpunkten innerhalb einer Schwellenanzahl von Basen von einer Vielzahl von Positionen aus umfassen.

4. Verfahren nach einem der vorhergehenden Ansprüche, wobei das Gruppieren der Kandidatenfusionssequenzlesungen basierend auf einem oder mehreren gemeinsamen Bruchpunkten das Erstellen eines de-Bruijn-Graphen für die Gruppen, zum Beispiel für jede Gruppe, umfasst; wobei optional das Zusammensetzen der Kandidatenfusionssequenzlesungen in den Gruppen zu einem oder mehreren Contigs das Linearisieren der de-Bruijn-Graphen umfasst, um ein Contig für die Gruppen zu erzeugen.

5. Verfahren nach einem der vorhergehenden Ansprüche, wobei das Zusammensetzen der Kandidatenfusionssequenzlesungen in den Gruppen zu einem oder mehreren Contigs das Durchführen einer oder mehrerer Fehlerkorrekturprozeduren umfasst; beispielsweise wobei die eine oder die mehreren Fehlerkorrekturprozeduren umfassen
(i) Auflösen von Fehlpaarungen zwischen Kandidatenfusionssequenzlesungen und der Referenzsequenz;
(ii) Einfügen von Lückenfüllung zwischen mindestens zwei Kandidatenfusionssequenzlesungen; und/oder
(iii) Verwerfen einer oder mehrerer Kandidatenfusionssequenzlesungen, die einen nichtalignierten Teil aufweisen, der einen Schwellenwert überschreitet.

6. Verfahren nach einem der vorhergehenden Ansprüche, wobei das Alignieren der Contigs aus den Gruppen der Vielzahl von Gruppen an der Referenzsequenz das Alignieren an eine Referenzsequenz mit Decoys (Ködersequenzen) umfasst.

7. Verfahren nach einem der vorhergehenden Ansprüche, wobei eine Familie basierend auf molekularer Barcode-Markierung bestimmt wird, beispielsweise wobei
(i) Kandidatenfusionssequenzlesungen, die den gleichen molekularen Barcode enthalten, in dieselbe Familie gruppiert werden; und/oder
(ii) Sequenzlesungen, die den gleichen molekularen Barcode enthalten und deren Alignments innerhalb einer Anzahl von Basen, beispielsweise 30-50 Basen, voneinander beginnen, in dieselbe Familie gruppiert werden.

8. Verfahren nach einem der vorhergehenden Ansprüche, wobei das Bestimmen, basierend auf den Alignments der Contigs aus den Gruppen, eines oder mehrerer Kandidatenfusionsereignisse ferner das Anwenden eines Footprint-Tests umfasst, wobei das Anwenden des Footprint-Tests das Bestimmen umfasst, dass eine Schwellenanzahl von Familien von Kandidatenfusionssequenzlesungen, die das Contig stützen, die Bruchpunkt (e) überspannen; wobei die Schwelle beispielsweise irgendwo von 2 bis 5 Familien, jeweils inklusive, liegt.

9. Verfahren nach einem der vorhergehenden Ansprüche, wobei der Schwellenwert für den Spread-Test sich auf 8 Basen, 7 Basen, 6 Basen oder 5 Basen beläuft.

10. Verfahren nach einem der vorhergehenden Ansprüche, wobei das Anwenden eines oder mehrerer Kriterien auf das eine oder die mehreren Kandidatenfusionsereignisse Folgendes umfasst:
(i) Bestimmen, für die Kandidatenfusionsereignisse, eines Abstands zwischen einem Bruchpunkt des einen oder der mehreren alignierten Contigs und einem Ort mindestens einer Sonde eines Panels; und
Verwerfen jedes Kandidatenfusionsereignisses, das mit einem alignierten Contig von dem einen oder den mehreren Contigs assoziiert ist, das keinen Bruchpunkt mit einem Abstand von dem Ort mindestens einer Sonde eines Panels, der kleiner als ein Schwellenwert ist, enthält;
(ii) Bestimmen eines oder mehrerer Gene von Interesse; und
Verwerfen jedes Kandidatenfusionsereignisses, das mit einem alignierten Contig von dem einen oder den mehreren Contigs assoziiert ist, das keinen Bruchpunkt enthält, der mit dem einen oder den mehreren Genen von Interesse assoziiert ist;
(iii) Bestimmen, für die Kandidatenfusionsereignisse, dass ein Bruchpunkt von dem einen oder den mehreren alignierten Contigs eine Deletion ist; und
Verwerfen jedes Kandidatenfusionsereignisses, das mit einem alignierten Contig von dem einen oder den mehreren alignierten Contigs assoziiert ist, das eine Deletion innerhalb einer Anzahl von Basen beabstandet zu einer anderen Deletion liegend umfasst;
(iv) Bestimmen, für die Kandidatenfusionsereignisse, dass ein Bruchpunkt von dem einen oder den mehreren alignierten Contigs eine Deletion ist; und
Verwerfen jedes Kandidatenfusionsereignisses, das mit einem alignierten Contig von dem einen oder den mehreren Contigs assoziiert ist, das eine Deletion umfasst, die eine Anzahl an Basen umfasst, die kleiner als eine Schwelle ist;
(v) Verwerfen jedes Kandidatenfusionsereignisses, das mit einem alignierten Contig von dem einen oder den mehreren Contigs assoziiert ist, das eine Insertion oder eine Deletion umfasst, die vollständig in einer intronischen Region eingebettet ist;
(vi) Bestimmen, für das Kandidatenfusionsereignis, für den einen oder die mehreren alignierten Contigs, eines Verhältnisses von Molekülen zu Lesungen; und
Verwerfen jedes Kandidatenfusionsereignisses, das mit einem alignierten Contig von dem einen oder den mehreren Contigs assoziiert ist, das mit einem Verhältnis von Molekülen zu Lesungen größer als eine Schwelle assoziiert ist, und das nicht mit einem doppelsträngigen Stützmolekül assoziiert ist;
(vii) Bestimmen, für das Kandidatenfusionsereignis, für die Paare von Bruchpunkten des einen oder der mehreren alignierten Contigs, einer Sequenz, die an den Bruchpunkten des Paars von Bruchpunkten anliegt;
Alignieren der Sequenzen, die an den Bruchpunkten des Paars von Bruchpunkten anliegen;
Bestimmen einer Alignmentbewertung für das Alignment der Sequenzen, die an den Bruchpunkten des Paars von Bruchpunkten anliegen; und
Verwerfen jedes Kandidatenfusionsereignisses, das mit einem alignierten Contig von dem einen oder den mehreren Contigs assoziiert ist, basierend darauf, dass die Alignmentbewertung eine Schwelle überschreitet; und/oder
(viii) Bestimmen, für die Kandidatenfusionsereignisse, für die Paare von Bruchpunkten des einen oder der mehreren alignierten Contigs, einer Sequenz, die auf den Bruchpunkten des Paars von Bruchpunkten zentriert ist;
Alignieren der um die Bruchpunkte herum zentrierten Sequenzen gegeneinander;
Bestimmen einer Alignmentbewertung für das Alignment der um die Bruchpunkte herum zentrierten Sequenzen; und
Verwerfen jedes Kandidatenfusionsereignisses, das mit einem alignierten Contig von dem einen oder den mehreren Contigs assoziiert ist, basierend darauf, dass die Alignmentbewertung eine Schwelle überschreitet.

11. Verfahren nach einem der vorhergehenden Ansprüche, ferner umfassend das Erstellen eines Berichts, wobei zum Beispiel die Fusionsereignisse in dem Bericht angezeigt werden und/oder wobei der Bericht Diagnoseinformationen oder therapeutische Empfehlungen basierend auf der Bestimmung der Fusionsereignisse umfasst.

12. Vorrichtung, die Folgendes umfasst:
einen oder mehrere Prozessoren; und
Speicher, der prozessorausführbare Anweisungen speichert, die, wenn sie durch den einen oder die mehreren Prozessoren ausgeführt werden, bewirken, dass die Vorrichtung die Verfahren nach einem der Ansprüche 1-11 durchführt.

13. Nichtflüchtiges computerlesbares Medium, das prozessorausführbare Anweisungen speichert, die bei Ausführung durch mindestens eine Computervorrichtung bewirken, dass die mindestens eine Computervorrichtung die Verfahren nach einem der Ansprüche 1-11 durchführt.

14. System, das mindestens eine Computervorrichtung umfasst, die dazu ausgelegt ist, die Verfahren nach einem der Ansprüche 1-11 durchzuführen.

## Revendications

1. Procédé mis en œuvre par ordinateur comprenant :
aligner une pluralité de lectures de séquence par rapport à une séquence de référence ;
déterminer un ou plusieurs points d'arrêt dans un alignement d'une pluralité de lectures de séquence de la pluralité de lectures de séquence par rapport à la séquence de référence ;
identifier toutes lectures de séquence associées au ou aux points d'arrêt dans l'alignement comme lectures de séquence de fusion candidates ;
déterminer des lectures de séquence de fusion candidates associées à des points d'arrêt communs d'un ou plusieurs points d'arrêt ;
grouper les lectures de séquence de fusion candidates en fonction d'un ou de plusieurs points d'arrêt communs ;
assembler les lectures de séquence de fusion candidates dans les groupes en un ou plusieurs contigs ;
aligner les contigs des groupes de la pluralité de groupes par rapport à la séquence de référence ;
déterminer, sur la base des alignements des contigs des groupes, un ou plusieurs événements de fusion candidats,
la détermination comprenant :
aligner des lectures de séquences de fusion candidates pour le groupe par rapport au contig, regrouper les lectures de séquences de fusion candidates en familles de lectures associées à la même molécule, et appliquer un test d'étalement aux alignements ; dans lequel l'application du test d'étalement comprend la détermination qu'une quantité seuil d'étalement existe entre les lectures de séquences d'au moins deux familles de lectures de séquences de fusion candidates qui supportent le contig et couvrent le ou les points d'arrêt ; dans lequel l'étalement est défini comme l'écart type minimum, ou le plus bas, entre un écart type de points de début de lecture sur le contig et un écart type des points d'arrêt de lecture sur le contig ;
appliquer un ou plusieurs critères au ou aux événements de fusion candidats ; et
déterminer, en fonction de l'application du ou des critères au ou aux événements de fusion candidats, un ou plusieurs événements de fusion.

2. Procédé selon la revendication 1, dans lequel l'identification de quelconques lectures de séquences associées aux un ou plusieurs points d'arrêt dans l'alignement comme lectures de séquences de fusion candidates comprend
(i) rejeter des alignements qui ont un score de mappabilité inférieur à un seuil ; et/ou
(ii) éliminer des alignements qui sont logiques.

3. Procédé selon la revendication 1 ou la revendication 2, dans lequel la détermination de lectures de séquences de fusion candidates associées à des points d'arrêt communs d'un ou plusieurs points d'arrêt comprend
(i) déterminer qu'au moins deux lectures de séquence de fusion candidates comprennent un point d'arrêt dans un même chromosome et à une même orientation ;
(ii) déterminer qu'au moins deux lectures de séquence de fusion candidates comprennent un point d'arrêt à une même position ;
(iii) déterminer qu'au moins deux lectures de séquences de fusion candidates comprennent un point d'arrêt à l'intérieur d'un nombre seuil de bases à partir d'une position ;
(iv) déterminer qu'au moins deux lectures de séquence de fusion candidates comprennent une pluralité de points d'arrêt dans un même chromosome et à une même orientation ;
(v) déterminer qu'au moins deux lectures de séquence de fusion candidates comprennent une pluralité de points d'arrêt aux mêmes positions ; et/ou
(vi) déterminer qu'au moins deux lectures de séquences de fusion candidates comprennent chacune une pluralité de points d'arrêt à l'intérieur d'un nombre seuil de bases à partir d'une pluralité de positions.

4. Procédé selon l'une quelconque des revendications précédentes, dans lequel le regroupement des lectures de séquence de fusion candidates sur la base d'un ou plusieurs points d'arrêt communs comprend la génération d'un graphe de Bruijn pour les groupes, par exemple pour chaque groupe ; facultativement, dans lequel l'assemblage des lectures de séquence de fusion candidates dans les groupes en un ou plusieurs contigs comprend la linéarisation des graphes de Bruijn pour générer un contig pour les groupes.

5. Procédé selon l'une quelconque des revendications précédentes, dans lequel l'assemblage des lectures de séquence de fusion candidates dans les groupes en un ou plusieurs contigs comprend l'exécution d'une ou plusieurs procédures de correction d'erreur ; par exemple dans lequel la ou les procédures de correction d'erreur comprennent
(i) résolution de mésappariements entre des lectures de séquence de fusion candidates et la séquence de référence ;
(ii) insertion d'un remplissage entre au moins deux lectures de séquence de fusion candidates ; et/ou
(iii) rejet d'une ou plusieurs séquences de fusion candidates ayant une partie non alignée qui dépasse un seuil.

6. Procédé selon l'une quelconque des revendications précédentes, dans lequel l'alignement des contigs des groupes de la pluralité de groupes par rapport à la séquence de référence comprend l'alignement par rapport à une séquence de référence avec des leurres.

7. Procédé selon l'une quelconque des revendications précédentes, dans lequel une famille est déterminée sur la base d'un codage à barres moléculaire, par exemple dans lequel
(i) les lectures de séquence de fusion candidates contenant le même code à barres moléculaire sont groupées dans la même famille ; et/ou
(ii) les lectures de séquence contenant le même code à barres moléculaire et dont des alignements commencent à l'intérieur d'un certain nombre de bases, par exemple 30-50 bases, sont regroupées dans la même famille.

8. Procédé selon l'une quelconque des revendications précédentes, dans lequel la détermination, sur la base des alignements des contigs des groupes, d'un ou plusieurs événements de fusion candidats comprend en outre l'application d'un test d'empreinte, dans lequel l'application du test d'empreinte comprend la détermination qu'un nombre seuil de familles de lectures de séquence de fusion candidates qui supportent le contig couvrent le ou les points d'arrêt ; par exemple dans lequel le seuil est n'importe où parmi, et inclut, 2 à 5 familles.

9. Procédé selon l'une quelconque des revendications précédentes, dans lequel le seuil pour le test d'étalement est de 8 bases, 7 bases, 6 bases ou 5 bases.

10. Procédé selon l'une quelconque des revendications précédentes, dans lequel l'application d'un ou plusieurs critères aux un ou plusieurs événements de fusion candidats comprend :
(i) déterminer, pour les événements de fusion candidats, une distance entre un point d'arrêt du ou des contigs alignés et un emplacement d'au moins une sonde d'un panel ; et
rejeter tout événement de fusion candidat associé à un contig aligné du ou des contigs ne contenant pas de point d'arrêt avec une distance de l'emplacement d'au moins une sonde d'un panel inférieure à un seuil ;
(ii) déterminer un ou plusieurs gènes d'intérêt ; et
rejeter tout événement de fusion candidat associé à un contig aligné du ou des contigs ne contenant pas de point d'arrêt qui est associé au ou aux gènes d'intérêt ;
(iii) déterminer, pour les événements de fusion candidats, qu'un point d'arrêt du ou des contigs alignés est une délétion ; et
rejeter tout événement de fusion candidat associé à un contig aligné du ou des contigs comprenant une délétion située dans un certain nombre de bases éloignées d'une autre délétion ;
(iv) déterminer, pour les événements de fusion candidats, qu'un point d'arrêt du ou des contigs alignés est une délétion ; et
rejeter tout événement de fusion candidat associé à un contig aligné du ou des contigs comprenant une délétion comprenant un nombre de bases inférieur à un seuil ;
(v) rejeter tout événement de fusion candidat associé à un contig aligné du ou des contigs comprenant une insertion ou une délétion qui est complètement incorporée dans une région intronique ;
(vi) déterminer, pour l'événement de fusion candidat, pour le ou les contigs alignés, un rapport de molécules sur lectures ; et
rejeter tout événement de fusion candidat associé à un contig aligné du ou des contigs qui est associé à un rapport de molécules sur lectures supérieur à un seuil et qui n'est pas associé à une molécule de support double brin ;
(vii) déterminer, pour l'événement de fusion candidat, pour les paires de points d'arrêt du ou des contigs alignés, une séquence aboutant aux points d'arrêt du couple de points d'arrêt ;
aligner les séquences aboutant aux points d'arrêt de la paire de points d'arrêt ;
déterminer un score d'alignement pour l'alignement des séquences aboutant aux points d'arrêts de la paire de points d'arrêts ; et
rejeter tout événement de fusion candidat associé à un contig aligné du ou des contigs sur la base du score d'alignement dépassant un seuil ; et/ou
(viii) déterminer, pour les événements de fusion candidats, pour les paires de points d'arrêt du ou des contigs alignés, une séquence centrée sur les points d'arrêt du couple de points d'arrêt ;
aligner les séquences centrées autour des points d'arrêt les unes par rapport aux autres ;
déterminer un score d'alignement pour l'alignement des séquences centrées autour des points d'arrêts ; et
rejeter tout événement de fusion candidat associé à un contig aligné du ou des contigs sur la base du score d'alignement dépassant un seuil.

11. Procédé selon l'une quelconque des revendications précédentes, comprenant en outre la génération d'un rapport, par exemple dans lequel les événements de fusion sont affichés dans le rapport et/ou dans lequel le rapport comprend des informations diagnostiques ou des recommandations thérapeutiques basées sur la détermination des événements de fusion.

12. Appareil comprenant :
un ou plusieurs processeurs ; et
une mémoire stockant des instructions exécutables par un processeur qui, lorsqu'elles sont exécutées par le ou les processeurs, amènent l'appareil à réaliser les procédés de l'une quelconque des revendications 1 à 11.

13. Support lisible par ordinateur non transitoire stockant des instructions exécutables par un processeur qui, lorsqu'elles sont exécutées par au moins un dispositif informatique, amènent l'au moins un dispositif informatique à réaliser les procédés selon l'une quelconque des revendications 1 à 11.

14. Système comprenant au moins un dispositif informatique configuré pour réaliser les procédés selon l'une quelconque des revendications 1 à 11.
